# EUROPÄISCHE PATENTSCHRIFT

(11) **EP 3 999 823 B1**
(45) Veröffentlichungstag und Bekanntmachung des Hinweises auf die Patenterteilung: **06.09.2023**
(21) Anmeldenummer: 20743103.2
(22) Anmeldetag: 15.07.2020
(51) Int. Cl.: G01J 3/02, G01N 21/3504

(54) **NICHTDISPERSIVE MEHRKANAL-SENSORBAUGRUPPE MIT REFRAKTIVEM STRAHLENTEILER**
NON-DISPERSIVE MULTI-CHANNEL SENSOR ASSEMBLY HAVING REFRACTIVE BEAMSPLITTER
ENSEMBLE DE CAPTEURS MULTICANAUX NON DISPERSIFS AVEC SÉPARATEUR DE FAISCEAUX RÉFRACTIF

(30) Priorität: 18.07.2019 DE 102019119502
(43) Veröffentlichungstag der Anmeldung: 25.05.2022
(73) Patentinhaber: Hamilton Medical AG, 7402 Bonaduz (CH)
(72) Erfinder: SCHRANZ, Christoph, 7306 Fläsch (CH); NOVOTNI, Dominik, 7000 Chur (CH); OFFENBECK, Bernd, 93057 Regensburg (DE)
(74) Vertreter: Ruttensperger Lachnit Trossin Gomoll
(86) Internationale Anmeldenummer: PCT/EP2020/069985
(87) Internationale Veröffentlichungsnummer: WO 2021/009216

(56) Entgegenhaltungen:
- EP-A1- 3 511 697
- DE-A1-102012 216 210
- DE-A1-102015 011 810
- US-A1- 2004 206 906

## Beschreibung

Die vorliegende Anmeldung betrifft eine nichtdispersive Mehrkanal-Strahlungssensorbaugruppe zur quantitativen Ermittlung eines eine elektromagnetische Messstrahlung absorbierenden Bestandteils eines Messfluids, umfassend:
- eine Strahlenteileranordnung, welche dazu ausgebildet ist, einen auf die Strahlenteileranordnung längs einer vorbestimmten Einstrahlachse einstrahlenden Strahl der Messstrahlung zu teilen,
- einen von einem ersten Teil der Messstrahlung erreichbaren ersten Bandfilter mit einer vorbestimmten ersten Bandbreite und mit einem Transmissionsmaximum bei einer vorbestimmten ersten Nutzsignal-Wellenlänge,
- einen im Strahlengang hinter dem ersten Bandfilter angeordneten ersten Messstrahlung-Nutzsignal-Sensor, auf welchen den ersten Bandfilter durchstrahlende Messstrahlung einstrahlt,
- einen räumlich vom ersten Bandfilter entfernt angeordneten zweiten Bandfilter, welcher von einem vom ersten Teil verschiedenen zweiten Teil der Messstrahlung erreichbar ist, wobei der zweite Bandfilter eine vorbestimmte zweite Bandbreite und ein Transmissionsmaximum bei einer vorbestimmten ersten Referenzsignal-Wellenlänge aufweist, wobei die erste Referenzsignal-Wellenlänge von der ersten Nutzsignal-Wellenlänge verschieden ist, und
- einen im Strahlengang hinter dem zweiten Bandfilter und räumlich vom ersten Messstrahlung-Nutzsignal-Sensor entfernt angeordneten ersten Messstrahlung-Referenzsignal-Sensor, auf welchen den zweiten Bandfilter durchstrahlende Messstrahlung einstrahlt.

Eine Mehrkanal-Strahlungssensorbaugruppe der eingangs genannten Art ist aus der US 2007/0241280 A1 als Infrarot-Sensorbaugruppe bekannt. Aus dieser Druckschrift ist es ebenfalls bekannt, eine solche Sensorbaugruppe zur Messung eines Gasanteils, insbesondere CO₂, in einem Beatmungsgas eines lebenden Patienten zu verwenden.

Bei der künstlichen Beatmung von lebenden Patienten, sei es nun eine künstliche Beatmung eines vollständig sedierten oder komatösen und damit zu eigener Atmung unfähigen Patienten oder sei es zur lediglich unterstützenden Beatmung eines wenigstens zeitabschnittsweise spontan atmenden Patienten, ist die Kenntnis von Gasbestandteilen des Beatmungsgases zur Überwachung der vitalen Funktionen des Patienten oder/und zur Überwachung des korrekten Betriebs der Beatmungsvorrichtung hilfreich und wichtig. Dies ist im einschlägigen Fachgebiet hinreichend bekannt. So kann beispielsweise durch Erfassung des CO₂-Anteils im inspiratorischen Beatmungsgas und im exspiratorischen Beatmungsgas ermittelt werden, wie gut die Verstoffwechselung von Sauerstoff im Patienten funktioniert. Dies soll jedoch nur eines von mehreren möglichen Beispielen sein.

Die aus der US 2007/0241280 A1 bekannte Gassensorbaugruppe verwendet einen sowohl reflektierenden als auch transmittierenden Strahlenteiler, um einen in den Strahlenteiler als Messstrahl einstrahlenden Infrarotstrahl (IR-Strahl) in zwei Infrarot-Teilstrahlen zu teilen. Ein erster Infrarot-Teilstrahl wird durch einen ersten Bandfilter auf einen ersten Sensor gelenkt. Dieser erste Bandfilter weist ein Transmissionsmaximum bei der Infrarot(IR)-Absorptions-Wellenlänge von CO₂ als der Nutzsignal-Wellenlänge auf und weist eine kleine erste Bandbreite auf, damit sich das Sensorsignal in Abhängigkeit vom jeweiligen CO₂-Gehalt des von dem in der bekannten Gassensorbaugruppe einstrahlenden Infrarotstrahl durchstrahlten, nachfolgend auch als "Messgas" bezeichneten Messfluids möglichst stark ändert. Der erste Sensor ist somit ein Messstrahlung- bzw. Infrarot-Nutzsignal-Sensor.

Ein zweiter Infrarot-Teilstrahl wird nach Durchgang durch einen zweiten Bandfilter und zusätzlich durch einen Kerbfilter auf einen zweiten Sensor gelenkt. Der zweite Bandfilter hat ein Transmissionsmaximum bei der Infrarot-Absorptionswellenlänge von CO₂ als der Referenzsignal-Wellenlänge und weist eine größere Bandbreite als der erste Bandfilter auf. Der Kerbfilter hat ein Auslöschungsmaximum bzw. ein Transmissionsminimum ebenfalls bei der Infrarot-Absorption-Wellenlänge von CO₂. Die Folge ist, dass sich das Signal des zweiten Sensors mit der Änderung des CO₂-Gehalts des Messgases nicht oder nur in vernachlässigbarem Umfang ändert. Der zweite Sensor ist folglich ein Messstrahlung- bzw. Infrarot-Referenzsignal-Sensor.

Der IR-Referenzsignal-Sensor ist erforderlich, um durch Vergleich des Signals des IR-Nutzsignal-Sensors mit dem Signal des IR-Referenzsignal-Sensors das Ausmaß an Absorption von Infrarotlicht durch CO₂ im Messgas und damit den Anteil von CO₂ im Messgas beurteilen zu können. Dies gilt auch für die vorliegende Erfindung, und zwar unabhängig davon, ob als Messstrahlung, wie es bevorzugt ist, eine IR-Strahlung oder eine elektromagnetische Strahlung anderer Wellenlänge verwendet wird.

Dabei ist es hilfreich, das Nutzsignal des IR-Nutzsignal-Sensors einerseits und das Referenzsignal des IR-Referenzsignal-Sensors aus ein und demselben einstrahlenden Infrarotstrahl abzuleiten, um sicherzustellen, dass sowohl das Nutzsignal als auch das Referenzsignal sowohl qualitativ als auch quantitativ im Wesentlichen denselben Störfaktoren ausgesetzt ist, sodass eine lediglich durch Störfaktoren bewirkte quantitative Änderung des Nutzsignals auch eine entsprechende Änderung des Referenzsignals bewirkt. Dadurch kann verhindert werden, dass aus einer Änderung des Nutzsignals irrtümlich auf eine Änderung des durch die Nutzsignal-Wellenlänge identifizierten Gasanteils geschlossen wird. Auch dies gilt für die vorliegende Erfindung.

In den in der der US 2007/0241280 A1 gezeigten schematischen Darstellungen sind die jeweiligen ebenen Sensor-Erfassungsflächen relativ zueinander um 90° geneigt orientiert. Die Sensor-Erfassungsfläche des IR-Nutzsignal-Sensors und die Sensor-Erfassungsfläche des IR-Referenzsignal-Sensors sind bezüglich einer die virtuelle Neigeachse enthaltenden Symmetrieebene spiegelsymmetrisch angeordnet.

Die US 2007/0241280 A1 nennt keine konkreten IR-Sensoren, die in der bekannten Gassensorbaugruppe zum Einsatz kommen. Es gibt jedoch IR-Sensoren, die nicht nur sensitiv für Einstrahlung von Infrarotlicht sind, sondern auch sensitiv für mechanische Belastungen, wie etwa Erschütterungen. Derartige mechanische sensitive IR-Sensoren sind überdies richtungsabhängig mechanisch sensitiv, d. h. eine betragsgleiche mechanische Belastung wirkt sich auf ein und denselben IR-Sensor abhängig von der Richtung, aus der sie auf den IR-Sensor einwirkt, unterschiedlich aus.

Nachteilig an der aus der US 2007/0241280 A1 bekannten Sensorbaugruppe ist daher, dass eine mechanische Belastung, die einheitlich auf die Sensorbaugruppe insgesamt einwirkt, sich in unterschiedlicher Weise auf den IR-Nutzsignal-Sensor und den IR-Referenzsignal-Sensor auswirkt und somit eine unerwünschte Ungenauigkeit in der Bestimmung des interessierenden Gasanteils im Messgas bewirken kann.

Nachteilig an der aus der US 2007/0241280 A1 bekannten Gassensorbaugruppe ist außerdem ihr Bauraumbedarf, um den Strahlenteiler und in den von diesem ausgehenden Teilstrahlengängen die genannten Filter und Sensoren anzuordnen.

Aus der EP 3 511 697 A1 ist eine nichtdispersive Strahlungssensorbaugruppe bekannt, deren Strahlenteileranordnung auf sie einfallende Strahlung beugend mittels eines computer-generierten Hologramms in divergierende Teilstrahlen teilt. Ein dem Hologramm in Durchstrahlungsrichtung unmittelbar nachgelagertes weiteres Beugungselement, etwa eine Fresnelzonenlinse, fokussiert diffraktiv vom Hologramm her auf sie einfallendes Licht auf an räumlich unterschiedlichen Orten angeordnete Sensoren, denen jeweils optische Filter vorgelagert sind.

Es ist daher Aufgabe der vorliegenden Erfindung, die eingangs genannte Mehrkanal-Strahlungssensorbaugruppe sowohl möglichst unempfindlich gegen lokale Störungen der Messstrahlung als auch mit möglichst geringem Bauraum und mit möglichst geringem Gewicht anzuordnen.

Die vorliegende Erfindung löst die genannte Aufgabe durch eine nichtdispersive Mehrkanal-Strahlungssensorbaugruppe der eingangs genannten Art, bei welcher die Strahlenteileranordnung eine von der Messstrahlung durchstrahlte und die durchstrahlende Messstrahlung brechende Strahlenteileranordnung ist, wobei die Strahlenteileranordnung wenigstens einen ersten Einstrahlbereich und wenigstens einen räumlich vom ersten verschiedenen zweiten Einstrahlbereich auf weist. In diesen Einstrahlbereichen strahlt Messstrahlung auf die Strahlenteileranordnung ein, wobei der erste und der zweite Einstrahlbereich optisch derart ausgebildet sind, dass
- die Strahlenteileranordnung im ersten Einstrahlbereich
   + einen ersten Teil der auf den ersten Einstrahlbereich einstrahlenden Messstrahlung auf den ersten Bandfilter ablenkt, und
   + einen zweiten Teil der auf den ersten Einstrahlbereich einstrahlenden Messstrahlung auf den zweiten Bandfilter ablenkt,
      und dass
- die Strahlenteileranordnung im zweiten Einstrahlbereich
   + einen ersten Teil der auf den zweiten Einstrahlbereich einstrahlenden Messstrahlung auf den zweiten Bandfilter ablenkt und
   + einen zweiten Teil der auf den zweiten Einstrahlbereich einstrahlenden Messstrahlung auf den ersten Bandfilter ablenkt,
wobei die Einstrahlbereiche wie in Anspruch 1 definiert ausgebildet sind.

Somit wird Messstrahlung, die auf die beiden Einstrahlbereiche einstrahlt, durch die Strahlenteileranordnung auf die beiden genannten Bandfilter und somit auf die den Bandfiltern zugeordneten Messstrahlung-Sensoren: erster Messstrahlung-Nutzsignal-Sensor und erster Messstrahlung-Referenzsignal Sensor, verteilt. Eine Störung, etwa durch Verschmutzung oder unerwünschte Fremdkörper im Strahlengang, welche sich nur lokal in einem der Einstrahlbereiche auf die Messstrahlung auswirkt, beeinflusst somit die Intensität der auf beide Bandfilter und folglich auf beide Messstrahlung-Sensoren ein strahlenden Teil-Messstrahlung nach Durchgang durch die Strahlenteileranordnung.

Grundsätzlich kann es ausreichen wenn nur jeweils ein Teil der einen Bandfilter durchstrahlende Messstrahlung auf den dem jeweiligen Bandfilter zugeordneten Messstrahlung-Sensor einfällt. Bevorzugt fällt die gesamte einen Bandfilter durchstrahlende Messstrahlung auf den dem jeweiligen Bandfilter zugeordneten Messstrahlung-Sensor ein, um einen möglichst hohen Signalpegel zu realisieren. Weiter bevorzugt fällt auf einen Messstrahlung-Sensor nur Strahlung ein, die zuvor den dem Messstrahlung-Sensor zugeordneten Bandfilter passiert hat.

Durch Brechung der die Strahlenteileranordnung durchstrahlenden Messstrahlung kann die oben definierte Aufteilung der Messstrahlung ausgehend von unterschiedlichen Einstrahlbereichen der Strahlenteileranordnung jeweils auf den ersten und den zweiten Bandfilter und folglich auf die zugeordneten Messstrahlung-Sensoren auf sehr kleinem Bauraum realisiert werden.

Die Mehrkanal-Strahlungssensorbaugruppe ist der Einfachheit halber nachfolgend auch nur als "Strahlungssensorbaugruppe" oder "Sensorbaugruppe" bezeichnet.

Der erste Einstrahlbereich kann optisch refraktiv wirksam sein und daher die ihn durchstrahlende Messstrahlung brechen. Um die oben genannte vorteilhafte Ablenkwirkung zu erzielen kann der erste Einstrahlbereich wenigstens zwei die einstrahlende Messstrahlung jeweils in unterschiedliche Richtungen ablenkende Ablenkzonen aufweisen. Dabei bewirkt bevorzugt wenigstens eine erste Ablenkzone die Ablenkung des ersten Teils der auf den ersten Einstrahlbereich einstrahlenden Messstrahlung auf den ersten Bandfilter. Wenigstens eine zweite Ablenkzone bewirkt bevorzugt die Ablenkung des zweiten Teils der auf den ersten Einstrahlbereich einstrahlenden Messstrahlung auf den zweiten Bandfilter. Die wenigstens eine erste und die wenigstens eine zweite Ablenkzone weisen unterschiedliches Ablenkverhalten bezüglich der Messstrahlung auf.

Zusätzlich oder alternativ kann der zweite Einstrahlbereich optisch refraktiv wirksam sein und wenigstens zwei die einstrahlende Messstrahlung jeweils in unterschiedliche Richtungen ablenkende Ablenkzonen aufweisen. Der zweite Einstrahlbereich ist als refraktiv wirksamer Einstrahlbereich analog zum oben beschriebenen refraktiv wirksamen ersten Einstrahlbereich ausgebildet. Folglich bewirkt wenigstens eine erste Ablenkzone die Ablenkung des ersten Teils der auf den zweiten Einstrahlbereich einstrahlenden Messstrahlung. Wenigstens eine zweite Ablenkzone bewirkt die Ablenkung des zweiten Teils der auf den zweiten Einstrahlbereich einstrahlenden Messstrahlung. Auch in einem refraktiv wirksamen zweiten Einstrahlbereich unterscheiden sich die wenigstens eine erste Ablenkzone und die wenigstens eine zweite Ablenkzone - wie im refraktiv wirksamen ersten Einstrahlbereich - durch ihr Ablenkverhalten bezüglich der Messstrahlung.

Die Unterschiede im Ablenkverhalten zwischen der wenigstens einen ersten und der wenigstens einen zweiten Ablenkzone eines Einstrahlbereichs, unabhängig davon ob es der erste oder der zweite Einstrahlbereich ist, können auf die Messstrahlung unterschiedlich brechende Materialien beruhen, aus welchen jeweils die wenigstens eine erste und die wenigstens eine zweite Ablenkzone gebildet sind. So können die wenigstens eine erste Ablenkzone und die wenigstens eine zweite Ablenkzone aus Materialien mit bezüglich der Messstrahlung unterschiedlichen Brechungsindizes gebildet sein.

Zusätzlich oder alternativ kann ein unterschiedliches Ablenkverhalten der genannten Ablenkzonen durch lokal unterschiedliche Grenzflächengestalten an einer die Strahlenteileranordnung im Bereich ihres Einstrahlbereichs von ihrer Umgebung scheidenden Grenzfläche begründet sein. Folglich kann die wenigstens eine erste Ablenkzone eine andere Grenzflächengestalt aufweisen und damit Messstrahlung anders ablenken als die wenigstens eine zweite Ablenkzone. Refraktiv wirkende optische Gebilde mit bereichs- bzw. zonenweise unterschiedlichen Grenzflächengestalten sind in der Optik beispielsweise von Fresnel-Linsen bekannt. Im Unterschied zu der vorliegenden refraktiven Strahlenteileranordnung, bei der in unterschiedlichen Zonen einfallende Messstrahlung austrittseitig zu unterschiedlichen Bandfiltern hin abgelenkt wird, weisen Fresnel-Linsen einen einheitlichen Brennpunkt auf und sind aus Gründen einer gewünschten Materialeinsparung zur Bildung dieser Linsen mit zonenweise unterschiedlicher Grenzflächengestalt ausgebildet.

Die wenigstens eine erste und die wenigstens eine zweite Ablenkzone weisen daher bevorzugt keine übereinstimmenden Brennpunkte oder/und keine einheitliche Brennweite auf. Für die Strahlenteileranordnung kommt es eher darauf an, auf sie einfallende Messstrahlung möglichst gleichmäßig auf mehrere Bandfilter zu verteilen, als darauf, die einfallende Messstrahlung möglichst exakt in einer Bildebene abzubilden.

Grundsätzlich ist es denkbar, die Bandfilter und die Strahlenteileranordnung mit ihren Einstrahlbereichen derart anzuordnen, dass jeder Einstrahlbereich von den beiden Bandfiltern, auf welche er jeweils Teile der einstrahlenden Messstrahlung ablenken soll, etwa gleich weit entfernt gelegen ist. Da eine solche Symmetriebedingung nicht stets mit den übrigen räumlichen Verhältnissen der Sensorbaugruppe in Einklang zu bringen ist, ist es zur möglichst flexiblen Anordnung von Strahlenteileranordnung und Bandfiltern sowie Messstrahlung-Sensoren bevorzugt, wenn die wenigstens eine erste und die wenigstens eine zweite Ablenkzone auf ihren Einstrahlbereich einstrahlende Messstrahlung bezüglich einer optischen Achse der Strahlenteileranordnung jeweils unterschiedlich stark ablenken. Im Zweifel, also sofern aufgrund der Brechungswirkung der Strahlenteileranordnung keine eindeutige optische Achse definierbar ist, soll die optische Achse die Strahlenteileranordnung zentral und orthogonal zu ihrer Haupterstreckungsfläche durchsetzen. Sofern auch hierbei Schwierigkeiten bei der Bestimmung einer optischen Achse bestehen, soll als optische Achse eine die Strahlenteileranordnung möglichst zentral durchsetzende Parallele zur Einstrahlrichtung der Messstrahlung auf die Strahlenteileranordnung herangezogen werden.

Grundsätzlich kann es ausreichen, dass der erste Einstrahlbereich nur genau eine erste und genau eine zweite Ablenkzone umfasst. Die Unempfindlichkeit gegenüber Störungen der Messstrahlung nimmt jedoch mit der Anzahl an ersten und zweiten Ablenkzonen zu, sodass bevorzugt der erste Einstrahlbereich eine Mehrzahl von ersten oder/und von zweiten Ablenkzonen umfasst. Aus den gleichen Gründen umfasst zusätzlich oder alternativ bevorzugt der zweite Einstrahlbereich eine Mehrzahl von ersten oder/und von zweiten Ablenkzonen.

Wenn ein Einstrahlbereich, sei es der erste oder/und der zweite Einstrahlbereich, eine Mehrzahl von ersten und zweiten Ablenkzonen aufweist, kann dieser Einstrahlbereich fertigungstechnisch dadurch mit möglichst geringem Aufwand hergestellt werden, dass, bei Betrachtung der Strahlenteileranordnung in einer zu einer optischen Achse der Strahlenteileranordnung parallelen oder die optische Achse enthaltenden Schnittebene durch die Strahlenteileranordnung, erste Ablenkzonen und zweite Ablenkzonen desselben Einstrahlbereichs alternierend aufeinander folgend angeordnet sind. Die ersten und die zweiten Ablenkzonen sind in der Schnittebene bevorzugt in einer Richtung von der optischen Achse weg aufeinander alternierend folgend angeordnet. Dies gilt bevorzugt für eine Mehrzahl von die optische Achse enthaltenden Schnittebenen, insbesondere für alle die optische Achse enthaltenden Schnittebenen. Dann kann die Strahlenteileranordnung bevorzugt rotationssymmetrisch mit der optischen Achse als Rotationssymmetrieachse ausgebildet sein. Im Falle, dass die oben genannte Bedingung für eine Mehrzahl von zur optischen Achse und zueinander parallelen Schnittebenen gilt, kann die Strahlenteileranordnung spiegelsymmetrisch bezüglich einer zur optischen Achse parallelen oder die optische Achse enthaltenden Spiegelsymmetrieebene sein. Die genannten symmetrischen Ausbildungen der Strahlenteileranordnung verringern die Fehleranfälligkeit einer Montage der Strahlenteileranordnung in einem Gehäuse der Sensorbaugruppe. Bei spiegelsymmetrischer Ausbildung kommt es auf eine Vertauschung der einen mit der anderen Seite der Strahlenteileranordnung nicht an; bei rotationssymmetrischer Ausbildung kommt es auf eine Winkelorientierung der Strahlenteileranordnung um die Rotationssymmetrieachse nicht an. Außerdem kann durch eine symmetrisch ausgebildete Strahlenteileranordnung auch austrittseitig ein symmetrisch ausgebildeter Abbildungsfleck erhalten werden, was eine vorteilhafte gleichmäßige Verteilung von Messstrahlung über mehrere Bandfilter und Messstrahlung-Sensoren erleichtert.

Wie oben bereits beschrieben wurde, können sich die erste und die zweite Ablenkzone eines Einstrahlbereichs voneinander durch lokal unterschiedliche Grenzflächengestalten an einer die Strahlenteileranordnung im Bereich ihres Einstrahlbereichs von ihrer Umgebung scheidenden Grenzfläche unterscheiden. Dabei hat es sich in Versuchen für die von der Strahlenteileranordnung ausgehende gebrochene Messstrahlung als vorteilhaft herausgestellt, wenn ein die erste Ablenkzone und die zweite Ablenkzone aufweisender Grenzflächenbereich eine Oberflächen-Einhüllende aufweist, deren parallel zu einer optischen Achse der Strahlenteileranordnung zu messender Abstand von einer zur optischen Achse orthogonalen Tangentialebene an die Grenzfläche mit zunehmender Entfernung von der optischen Achse der Strahlenteileranordnung größer wird. Dann kann auf die Strahlenteileranordnung einstrahlende Messstrahlung auf der Austrittsseite der Strahlenteileranordnung vorteilhaft gleichmäßig über einen ausreichend großen Flächenbereich verteilt abgelenkt werden, sodass zur Anordnung der Bandfilter und der zugeordneten Messstrahlung-Sensoren ausreichend Anordnungsfläche bereitsteht.

Die brechende Grenzfläche kann an der Strahlenteileranordnung auf der von den Bandfiltern und Messstrahlung-Sensoren abgewandten oder/und auf der entgegengesetzten, den Bandfiltern und Messstrahlung-Sensoren zugewandten Seite der Strahlenteileranordnung ausgebildet sein.

Bevorzugt ist die Oberflächen-Einhüllende bei Betrachtung der Strahlenteileranordnung von außen konvex gekrümmt, sodass ein refraktiv wirksamer Abschnitt, bevorzugt der gesamte refraktiv wirksame Abschnitt der Strahlenteileranordnung vollständig auf einer Seite der Tangentialebene gelegen ist. Hierdurch kann der für die Strahlenteileranordnung benötigte Bauraum vorteilhaft kleingehalten werden.

Als bevorzugt rotationssymmetrisch ausgebildete Strahlenteileranordnung kann die Oberflächen-Einhüllende eine konische, eine kegelstumpfförmige eine konvex oder eine konkav gekrümmte Gestalt aufweisen. Die konkav gekrümmte Gestalt ist dabei nicht bevorzugt, aber grundsätzlich möglich.

Wenngleich es grundsätzlich möglich ist, dass die refraktiv wirksame Strahlenteileranordnung aus unterschiedlichen gesondert hergestellten Teilanordnungen gebaut ist, ist es zur dauerhaften Sicherstellung der die Messstrahlung brechenden Eigenschaften der Strahlenteileranordnung vorteilhaft, wenn wenigstens eine erste Ablenkzone, bevorzugt wenigstens ein Teil der ersten Ablenkzonen, vorzugsweise alle erste Ablenkzonen, des ersten und des zweiten Einstrahlbereichs wenigstens abschnittsweise, vorzugsweise vollständig, einstückig zusammenhängend ausgebildet sind oder/und wenn wenigstens eine zweite Ablenkzone, bevorzugt wenigstens ein Teil der zweiten Ablenkzonen, vorzugsweise alle zweiten Ablenkzonen, des ersten und des zweiten Einstrahlbereichs wenigstens abschnittsweise, vorzugsweise vollständig, einstückig zusammenhängend ausgebildet sind.

Die obige nichtdispersive Mehrkanal-Messstrahlungssensorbaugruppe ist bisher am Beispiel einer Zweikanal-Messstrahlungssensorbaugruppe beschrieben und weitergebildet worden. Ausführungsformen der Sensorbaugruppe der vorliegenden Erfindung sind jedoch nicht auf die oben beschriebenen zwei Kanäle: ein Kanal für das erste Nutzsignal und ein weiterer Kanal für das erste Referenzsignal, beschränkt. Sie kann eine Drei-, Vier-, Fünf-, Sechs- oder Acht- oder noch höherzahlige Mehrkanal-Strahlungssensorbaugruppe sein, wobei für jeden Kanal ein Messstrahlung-Sensor und bevorzugt ein Filter, insbesondere Bandfilter, angeordnet ist. Weiter ist bevorzugt für jeden Kanal an der Strahlenteileranordnung ein eigener Einstrahlbereich vorgesehen, welcher auf ihn einstrahlende Messstrahlung austrittsseitig in wenigstens zwei unterschiedliche Richtungen zu unterschiedlichen Messstrahlung-Sensoren hin ablenkt.

Eine besonders bevorzugte Weiterbildung der oben beschriebenen nichtdispersiven Mehrkanal-Messstrahlungssensorbaugruppe kann zur Vergrößerung ihres Erfassungsumfangs zusätzlich umfassen:
- einen räumlich vom ersten und vom zweiten Bandfilter entfernt angeordneten, von einem dritten Teil der Messstrahlung erreichbaren dritten Bandfilter mit einer vorbestimmten dritten Bandbreite und mit einem Transmissionsmaximum bei einer vorbestimmten zweiten Nutzsignal-Wellenlänge,
- einen räumlich von dem ersten Messstrahlung-Nutzsignal-Sensor und dem ersten Messstrahlung-Referenzsignal-Sensor entfernt angeordneten, im Strahlengang hinter dem dritten Bandfilter angeordneten zweiten Messstrahlung-Nutzsignal-Sensor, auf welchen den dritten Bandfilter durchstrahlende Messstrahlung einstrahlt,
- einen räumlich vom ersten, zweiten und dritten Bandfilter entfernt angeordneten vierten Bandfilter, welcher von einem vom ersten, zweiten und dritten Teil verschiedenen vierten Teil der Messstrahlung erreichbar ist, wobei der vierte Bandfilter eine vorbestimmte vierte Bandbreite und ein Transmissionsmaximum bei einer vorbestimmten zweiten Referenzsignal-Wellenlänge aufweist, wobei die zweite Referenzsignal-Wellenlänge von der zweiten Nutzsignal-Wellenlänge verschieden ist,
- einen im Strahlengang hinter dem vierten Bandfilter und räumlich vom ersten und zweiten Messstrahlung-Nutzsignal-Sensor sowie vom ersten Messstrahlung-Referenzsignal-Sensor entfernt angeordneten zweiten Messstrahlung-Referenzsignal-Sensor, auf welchen den vierten Bandfilter durchstrahlende Messstrahlung einstrahlt,
wobei die Strahlenteileranordnung einen vom ersten und vom zweiten Einstrahlbereich räumlich verschiedenen dritten Einstrahlbereich sowie einen vom ersten, zweiten und dritten Einstrahlbereich räumlich verschiedenen vierten Einstrahlbereich aufweist, wobei in den dritten und vierten Einstrahlbereichen Messstrahlung auf die Strahlenteileranordnung einstrahlt, und wobei der dritte und der vierte Einstrahlbereich optisch derart ausgebildet sind, dass
- die Strahlenteileranordnung im dritten Einstrahlbereich
   + einen ersten Teil der auf den dritten Einstrahlbereich einstrahlenden Messstrahlung auf den dritten Bandfilter ablenkt, und
   + einen zweiten Teil der auf den dritten Einstrahlbereich einstrahlenden Messstrahlung auf den vierten Bandfilter ablenkt,
      und dass
- die Strahlenteileranordnung im vierten Einstrahlbereich
   + einen ersten Teil der auf den vierten Einstrahlbereich einstrahlenden Messstrahlung auf den vierten Bandfilter ablenkt und
   + einen zweiten Teil der auf den vierten Einstrahlbereich einstrahlenden Messstrahlung auf den dritten Bandfilter ablenkt.

Zur Vermeidung unnötiger Wiederholungen sei klargestellt: es gilt das oben, und ebenso unten, zum ersten Einstrahlbereich Gesagte mutatis mutandis für den dritten Einstrahlbereich entsprechend. Ebenso gilt das oben, und ebenso unten, zum zweiten Einstrahlbereich Gesagte mutatis mutandis für den vierten Einstrahlbereich entsprechend. Der Sammelbegriff "Messstrahlung-Sensor" erstreckt sich somit auch auf den zweiten Messstrahlung-Nutzsignal-Sensor sowie auf den zweiten Messstrahlung-Referenzsignal-Sensor. Weiterhin gilt das oben zum ersten Bandfilter und dem ersten Messstrahlung-Nutzsignal-Sensor Gesagte mutatis mutandis für den dritten Bandfilter und den zweiten Messstrahlung-Nutzsignal-Sensor entsprechend. Ebenso gilt das oben zum zweiten Bandfilter und dem ersten Messstrahlung-Referenzsignal-Sensor Gesagte mutatis mutandis für den vierten Bandfilter und den zweiten Messstrahlung-Referenzsignal-Sensor entsprechend.

Die erste Nutzsignal-Wellenlänge ist bevorzugt eine Wellenlänge oder ein Wellenlängenbereich der Messstrahlung, bei welcher bzw. in welchem Messstrahlung von einem zu erfassenden Bestandteil des Messfluids absorbiert wird. Gleiches gilt bevorzugt für die zweite Nutzsignal-Wellenlänge. Dabei kann dann die zweite Nutzsignal-Wellenlänge betragsmäßig von der ersten Nutzsignal-Wellenlänge verschieden sein. Der betragsmäßige Unterschied zwischen der ersten und der zweiten Nutzsignal-Wellenlänge kann erheblich sein, etwa dann wenn das Messfluid zwei unterschiedliche Messfluidbestandteile mit unterschiedlichen Absorptionswellenlängen aufweist, von welchen die erste Absorptionswellenlänge mit der ersten Nutzsignal-Wellenlänge des ersten Messstrahlung-Nutzsignal-Sensors und die zweite Absorptionswellenlänge mit einer zweiten Nutzsignal-Wellenlänge des zweiten Messstrahlung-Nutzsignal-Sensors übereinstimmt. Dann können durch eine einzige Mehrkanal-Strahlungssensorbaugruppe zwei unterschiedliche Messfluidbestandteile in ein und derselben Messphase erfasst werden.

Die Nutzsignal-Wellenlängen definieren, wie oben beschrieben, von der Strahlungssensorbaugruppe erfassbare Bestandteile im Messfluid. Grundsätzlich kann daran gedacht sein, dass sich die Nutzsignal-Wellenlängen betragsmäßig unterscheiden, obwohl sie denselben Messfluidbestandteil erfassen sollen. Um sicherstellen zu können, dass die Messstrahlung-Nutzsignal-Sensoren den auf sie einfallenden Messstrahlungsteil hinsichtlich der von diesem transportierten Absorptionsinformation optimal auswerten können, ist es vorteilhaft, wenn die Nutzsignal-Wellenlängen sich betragsmäßig um nicht mehr als ein Drittel der betragsmäßig kleineren Bandbreite aus erster und dritter Bandbreite unterscheiden. Bei Erfassung nur eines Bestandteils des Messfluids durch wenigstens zwei Messstrahlung-Nutzsignal-Sensoren mit jeweils unterschiedlichen Nutzsignal-Wellenlängen liegen die Nutzsignal-Wellenlängen bevorzugt betragsmäßig auf unterschiedlichen Seiten der Absorptionswellenlänge des Bestandteils. Bevorzugt unterscheiden sich die beiden Nutzsignal-Wellenlängen um nicht mehr als 2 %, bezogen auf die betragsmäßig kleinere der beiden Wellenlängen, besonders bevorzugt um nicht mehr als 1 %. Die erste und die zweite Nutzsignal-Wellenlänge können betragsmäßig gleich gewählt sein, um redundante Signale zu erhalten.

Dann, wenn sich die erste und die zweite Nutzsignal-Wellenlänge betragsmäßig unterscheiden, weist bevorzugt eine Absorptionswellenlänge des zu erfassenden Bestandteils des Messfluids einen Wert auf, welcher betragsmäßig zwischen der ersten und der zweiten Nutzsignal-Wellenlänge liegt, sodass sich bei Änderung der Menge an dem zu erfassenden Bestandteil im Messfluid und damit verbunden bei einer Änderung der Absorption der Messstrahlung im Messfluid die Nutzsignale sowohl des ersten als auch des zweiten Messstrahlung-Nutzsignal-Sensors ändern. Dann kann eine besonders fehlerunanfällige Erfassung ein und desselben Bestandteils des Messfluids mit der Mehrkanal-Strahlungssensorbaugruppe erfolgen.

Zur sauberen Trennung von Nutzsignal und Referenzsignal ist vorzugsweise jede Wellenlänge aus der ersten und der zweiten Referenzsignal-Wellenlänge von jeder Wellenlänge aus der ersten und der zweiten Nutzsignal-Wellenlänge verschieden. Sofern nur ein Messstrahlung-Nutzsignal-Sensor und ein Messstrahlung-Referenzsignal-Sensor vorgesehen sind, sind die erste Referenzsignal-Wellenlänge und die erste Nutzsignal-Wellenlänge voneinander verschieden. Durch die beschriebene Verschiedenheit kann sichergestellt werden, dass ein Nutzsignal durch Änderungen am Messfluid, etwa seiner Zusammensetzung, veränderbar ist, während ein zur Bestimmung des Maßes an Änderung des Nutzsignals notwendiges Referenzsignal durch die Änderungen am Messfluid möglichst unverändert und somit im Wesentlichen konstant bleibt. Dann kann das Referenzsignal als Maßstab für eine Bestimmung einer Änderung eines Nutzsignals dienen.

Grundsätzlich kann daran gedacht sein, dass jede der Referenzsignal-Wellenlängen größer als jede der Nutzsignal-Wellenlängen oder kleiner als jede der Nutzsignal-Wellenlängen ist. Grundsätzlich können auch beide Referenzsignal-Wellenlängen identisch sein. Eine besonders hohe Qualität des Referenzsignals kann man jedoch dann erhalten, wenn die beiden Referenzsignal-Wellenlängen betragsmäßig verschieden sind, da dann unerwünschte Zufallsstörungen durch einen vorübergehend unerwartet vorhandenen Bestandteil des Messfluids sich nur auf einen der beiden Messstrahlung-Referenzsignal-Sensoren auswirken können. Zur Bereitstellung eines möglichst robusten Referenzsignals ist die erste oder die zweite Nutzsignal-Wellenlänge betragsmäßig zwischen der ersten und der zweiten Referenzsignal-Wellenlänge gelegen. Besonders bevorzugt sind beide Nutzsignal-Wellenlängen zwischen der ersten und der zweiten Referenzsignal-Wellenlänge gelegen.

Um dafür zu sorgen, dass das Signal des wenigstens einen Messstrahlung-Nutzsignal-Sensors empfindlich auf Änderungen des Anteils des durch die Nutzsignal-Wellenlängen definierten, zu erfassenden Messfluidbestandteils reagiert, während das Signal der Messstrahlung-Referenzsignal-Sensoren möglichst konstant bleiben soll, ist es vorteilhaft, wenn jede Bandbreite aus zweiter und vierter Bandbreite betragsmäßig kleiner ist als jede Bandbreite aus erster und dritter Bandbreite. So können die zweiten und vierten Bandfilter jeweils eine Bandbreite im zweistelligen Nanometerbereich aufweisen und können der erste und der dritte Bandfilter jeweils eine Bandbreite im dreistelligen Nanometerbereich aufweisen. Von dem zweiten und dem vierten Bandfilter kann, ebenso wie vom ersten und dritten Bandfilter, derjenige Bandfilter mit der kleineren Transmissionsmaximum-Wellenlänge die größere Bandbreite aufweisen. Bandbreiten für den zweiten und vierten Bandfilter liegen bevorzugt in einem Bereich von 50 bis 99 nm, insbesondere in einem Bereich von 60 bis 90 nm. Bandbreiten für den ersten und dritten Bandfilter liegen bevorzugt im Bereich von 150 bis 200 nm, insbesondere in einem Bereich von 170 bis 180 nm.

Abweichend vom oben Gesagten kann dann, wenn die erste Referenzsignal-Wellenlänge ein sicheres und robustes Referenzsignal bietet, etwa weil sie fern von möglichen oder erwarteten Absorptionswellenlängen von jeweiligen Bestandteilen des Messfluids gelegen ist, lediglich ein Messstrahlung-Referenzsignal-Sensor ausreichen. Der oben genannte Messstrahlung-Referenzsignal-Sensor kann dann ein dritter Messstrahlung-Nutzsignal-Sensor sein. Somit kann entweder eine Erfassung des ersten oder/und des zweiten Messstrahlung-Nutzsignal-Sensors durch den dritten Messstrahlung-Nutzsignal-Sensor verifiziert werden, oder es kann durch geeignete Wahl des Transmissionsmaximums des dritten Bandfilters ein dritter Bestandteil des Messfluids durch die Mehrkanal-Strahlungssensorbaugruppe in ein und derselben Messphase erfasst werden.

Anstelle der oben genannten Komponenten: vierter Bandfilter und zweiter Messstrahlung-Nutzsignal-Sensor, weist die bevorzugte Ausführungsform der Mehrkanal-Strahlungssensorbaugruppe dann auf:
- einen räumlich vom ersten, zweiten und dritten Bandfilter entfernt angeordneten vierten Bandfilter, welcher von einem vom ersten, zweiten und dritten Teil verschiedenen vierten Teil der Messstrahlung erreichbar ist, wobei der vierte Bandfilter eine vorbestimmte vierte Bandbreite und ein Transmissionsmaximum bei einer vorbestimmten dritten Nutzsignal-Wellenlänge aufweist,
- einen im Strahlengang hinter dem vierten Bandfilter und räumlich vom ersten und zweiten Messstrahlung-Nutzsignal-Sensor sowie vom ersten Messstrahlung-Referenzsignal-Sensor entfernt angeordneten dritten Messstrahlung-Nutzsignal-Sensor, auf welchen den vierten Bandfilter durchstrahlende Messstrahlung einstrahlt.

Baulich sind die beiden Versionen der Strahlungssensorbaugruppe gleich. Lediglich das von der Kombination aus dem vierten Bandfilter und dem dem vierten Bandfilter zugeordneten Messstrahlung-Sensor gelieferte Signal wird einmal als Referenzsignal und einmal als Nutzsignal ausgewertet.

Die vorliegend vorgestellte Mehrkanal-Strahlungssensorbaugruppe ist bevorzugt eine nichtdispersive CO₂- oder/und eine nichtdispersive NOₓ-, insbesondere eine nichtdispersive NO₂-Strahlungssensorbaugruppe. Bevorzugt ist die Messstrahlung Infrarotstrahlung. Grundsätzlich kann die Messstrahlung aber jede andere Strahlung sein, deren Wellenlängenspektrum eine Absorptionswellenlänge eines zu erfassenden Bestandteils des Messfluid enthält.

Ein bevorzugter Anwendungsfall der vorliegend beschriebenen Strahlungssensorbaugruppe ist die Erfassung von CO₂ in inspiratorischem oder/und exspiratorischem Beatmungsgas während einer apparativen Beatmung eines Patienten. Die erste und die zweite Nutzsignal-Wellenlänge liegen zur CO₂-Erfassung bevorzugt in einem Bereich zwischen 4,25 µm und 4,28 µm. Die erste Referenzsignal-Wellenlänge kann im Bereich von 3,90 µm bis 4,0 µm, besonders bevorzugt bei 3,95 µm liegen, die zweite Referenzsignal-Wellenlänge kann im Bereich von 4,40 µm bis 4,5 µm, besonders bevorzugt bei 4,45 µm liegen.

Die vorliegende Erfindung ermöglicht eine besonders bauraumsparende Ausgestaltung der Mehrkanal-Strahlungssensorbaugruppe auch dadurch, dass eine Mehrzahl von Bandfiltern oder/und eine Mehrzahl von Messstrahlung-Sensoren jeweils in einer Bandfilter-Anordnungsebene bzw. in einer Sensor-Anordnungsebene angeordnet sein können. Dann sind die Bandfilter eben ausgebildet, wobei die ebenen Bandfilter mit ihrer Erstreckungsebene parallel zur Bandfilter-Anordnungsebene angeordnet. Ebenso weisen die Messstrahlung-Sensoren bevorzugt eine für Messstrahlung empfindliche Sensor-Erfassungsebene auf, wobei die Messstrahlung-Sensoren mit ihrer jeweiligen Sensor-Erfassungsebene parallel zur Sensor-Anordnungsebene angeordnet sind.

Bevorzugt sind die Bandfilter-Anordnungsebene, in der die Mehrzahl von Bandfiltern angeordnet ist, und die Sensor-Anordnungsebene, in welcher die Mehrzahl von Messstrahlung-Sensoren angeordnet sind, zueinander parallel. Ebenso bevorzugt sind zur Erzielung einer möglichst großen Ausleuchtung von Bandfiltern und Messstrahlung-Sensoren sowohl die Bandfilter-Anordnungsebene als auch die Sensor-Anordnungsebene orthogonal zur optischen Achse der Strahlenteileranordnung ausgerichtet. Bevorzugt sind alle Bandfilter in einer gemeinsamen Sensor-Anordnungsebene angeordnet. Ebenso sind bevorzugt alle Messstrahlung-Sensoren in einer gemeinsamen Sensor-Anordnungsebene angeordnet. Zur Erzielung effektiver Raumausnutzung können die Bandfilter oder/und die Messstrahlung-Sensoren in ihrer jeweiligen Anordnungsebene gekachelt angeordnet sein. Bevorzugt liegen sich dabei geradlinige Randabschnitte unterschiedlicher Bandfilter oder/und Messstrahlung-Sensoren in den jeweiligen Anordnungsebenen parallel gegenüber oder stoßen aneinander an. Um eine möglichst gute Raumausnutzung in der jeweiligen Anordnungsebene bereitstellen zu können, können die Bandfilter oder/und die Messstrahlung-Sensoren eine rechteckige oder hexagonale Grundfläche aufweisen.

Zusätzlich zu der oben beschriebenen strahlungsbrechenden Einstrahlbereichen der Strahlenteileranordnung kann der erste oder/und der zweite Einstrahlbereich und, sofern vorhanden, zusätzlich der dritte oder/und der vierte Einstrahlbereich strahlungsbeugende, also diffraktive Wirkung haben.

Gemäß einer vorteilhaften Weiterbildung der vorliegenden Erfindung kann daher vorgesehen sein, dass der erste Einstrahlbereich optisch diffraktiv wirksam ist und eine Beugungsstruktur aufweist, welche eine auf ihn einstrahlende Messstrahlung durch Beugung ablenkt. Dabei lenkt der erste Einstrahlbereich auf ihn einstrahlende Messstrahlung sowohl auf den ersten als auch auf den zweiten Bandfilter durch Beugung ab. Die Beugungsstruktur kann ein Beugungsgitter oder eine vergleichbare Beugungsstruktur mit beugender Wirkung, wie etwa Beugungsspalte, aufweisen. Besonders bevorzugt kann die Beugungsstruktur kostengünstig als durchstrahlbares Hologramm ausgebildet sein.

Zusätzlich kann im gleichen Sinne der zweite Einstrahlbereich optisch diffraktiv wirksam sein und eine Beugungsstruktur aufweisen, welche eine auf ihn einstrahlende Messstrahlung durch Beugung ablenkt. Dabei lenkt der zweite Einstrahlbereich auf ihn einstrahlende Messstrahlung sowohl auf den ersten als auch auf den zweiten Bandfilter ab. Auch für den zweiten Einstrahlbereich gilt, dass die Beugungsstruktur ein Beugungsgitter oder eine vergleichbare Beugungsstruktur mit beugender Wirkung aufweisen kann, bevorzugt etwa als durchstrahlbares Hologramm.

Um auf die Strahlenteileranordnung einstrahlende Messstrahlung in möglichst unterschiedliche Richtungen möglichst gleichmäßig beugend ablenken zu können, kann die Strahlenteileranordnung in Einstrahlrichtung hintereinander eine Mehrzahl von Beugungsstrukturen aufweisen, sodass von einer ersten Beugungsstruktur gebeugte Messstrahlung auf eine zweite Beugungsstruktur einstrahlt und erneut gebeugt wird. Vorzugsweise unterscheiden sich die Hauptbeugungsrichtungen von wenigstens zwei hintereinander angeordneten Beugungsstrukturen. Besonders bevorzugt sind die Hauptbeugungsrichtungen von wenigstens zwei hintereinander angeordneten Beugungsstrukturen zueinander orthogonal.

Ebenso kann eine wenigstens abschnittsweise diffraktiv wirkende Strahlenteileranordnung längs ihrer optischen Achse zwei oder mehr hintereinander angeordnete refraktive Bauteile aufweisen.

Außerdem ist grundsätzlich möglich, dass die Strahlenteileranordnung in Einstrahlrichtung hintereinander eine Beugungsstruktur und eine die Messstrahlung brechende Struktur, also eine diffraktive und eine refraktive Struktur aufweist, ohne dass die Reihenfolge der Nennung eine Reihenfolge der Anordnung anzeigen soll.

Bisher wurde die Empfangsseite der Strahlungssensorbaugruppe beschrieben, da diese besonders vorteilhaft ausgestaltet ist. Die beschriebene Strahlungssensorbaugruppe kann für sich alleine genommen als abgeschlossene Baugruppe erzeugt, transportiert und montiert werden. Um ein Erfassungssignal aus der beschriebenen Strahlungssensorbaugruppe zu erhalten, ist jedoch eine Messstrahlungsquelle erforderlich. Diese kann gesondert von der Strahlungssensorbaugruppe vorliegen. Bevorzugt wird jedoch eine ohne weitere Maßnahmen einsatzbereite Strahlungssensorbaugruppe bereitgestellt, sodass gemäß einer Weiterbildung der vorliegenden Erfindung die Mehrkanal-Strahlungssensorbaugruppe bevorzugt mit Abstand von der Strahlenteileranordnung eine Messstrahlungsquelle aufweist, welche dazu ausgebildet ist, Messstrahlung zur Strahlenteileranordnung hin auszustrahlen. Wie oben bereits beschrieben wurde, ist die Messstrahlungsquelle bevorzugt eine Infrarotstrahlungsquelle.

Für eine hohe Strahlungsausbeute an den Messstrahlung-Sensoren und damit für einen hohen Nutzsignalpegel ist es vorteilhaft, die Ablenkung der auf die Strahlenteileranordnung einstrahlenden Messstrahlung möglichst exakt vorhersagen zu können. Um dies zu erreichen ist bevorzugt die Messstrahlungsquelle dazu ausgebildet ist, kollimierte Messstrahlung auszustrahlen. Diese Ausbildung kann an der Messstrahlungsquelle selbst realisiert sein, etwa in dem ein Messstrahlung aussenden der Laser verwendet wird. Zusätzlich oder alternativ kann die Messstrahlungsquelle eine Kollimatoranordnung aufweisen, welche in an sich bekannter Weise eine refraktive Optik oder/und eine Blendenanordnung aufweisen kann.

Weiter kann zur möglichst einfachen Handhabung die Strahlungssensorbaugruppe eine Auswertevorrichtung umfassen, welche aus den Signalen des ersten Messstrahlung-Referenzsignal-Sensors eine Referenzinformation ermittelt, welche aus den Signalen des ersten Messstrahlung-Nutzsignal-Sensors eine Nutzinformation ermittelt, und welche aus einem Vergleich von Referenzinformation und Nutzinformation eine Information über einen Anteil eines durch die erste Nutzsignal-Wellenlänge identifizierten Messfluid-Bestandteils an einem mit der Messstrahlung bestrahlten Messfluid ausgibt. Weist die Strahlungssensorbaugruppe mehr als einen Referenzsignal-Sensor und mehr als einen Nutzsignal-Sensor auf, werden auch deren Signale durch die Auswertevorrichtung in der genannten Weise verarbeitet, abhängig davon welche weiteren Messstrahlung-Sensoren an der Strahlungssensorbaugruppe angeordnet sind.

Für den bevorzugten Einsatz der Strahlungssensorbaugruppe an einer Beatmungsvorrichtung zur künstlichen Beatmung von Lebewesen ist es bevorzugt, wenn die Mehrkanal-Strahlungssensorbaugruppe ein Sensorgehäuse aufweist mit einem ersten Kompartiment, an oder in welchem die Strahlenteileranordnung, der Messstrahlung-Nutzsignal-Sensor und der Messstrahlung-Referenzsignal-Sensor angeordnet sind, und mit einem räumlich vom ersten Kompartiment entfernt gelegenen zweiten Kompartiment, an oder in welchem die Messstrahlungsquelle angeordnet ist, wobei zwischen dem ersten und dem zweiten Kompartiment eine Aufnahmeformation zur Aufnahme einer Messküvette zwischen dem ersten und dem zweiten Kompartiment angeordnet ist. Dann ist die Strahlungssensorbaugruppe räumlich und baulich von dem durch sie zu erfassenden Messfluid getrennt bzw. trennbar, sodass jegliche Art von Kontamination der Strahlungssensorbaugruppe durch das Messfluid in einem möglicherweise klinischen Betrieb ausgeschlossen oder wenigstens minimiert ist.

Aufgrund der bevorzugten Verwendung der oben genannten Mehrkanal-Strahlungssensorbaugruppe betrifft die vorliegende Erfindung auch eine Beatmungsvorrichtung zur wenigstens unterstützend künstlichen Beatmung eines lebenden Patienten, umfassend:
- eine Beatmungsgasquelle,
- eine Beatmungsleitungsanordnung, um inspiratorisches Beatmungsgas von der Beatmungsgasquelle zu einer patientenseitigen, proximalen Beatmungsgas-Auslassöffnung und um exspiratorisches Beatmungsgas von einer proximalen Beatmungsgas-Einlassöffnung weg zu leiten,
- eine Druckveränderungsvorrichtung zur Veränderung des Drucks des Beatmungsgases in der Beatmungsleitungsanordnung,
- eine Steuervorrichtung zum Betrieb der Beatmungsgasquelle oder/und der Druckveränderungsvorrichtung sowie
- eine Mehrkanal-Strahlungssensorbaugruppe nach einem der vorhergehenden Ansprüche zur Erfassung wenigstens eines Gasbestandteils im inspiratorischen oder/und exspiratorischen Beatmungsgas.

Die Beatmungsgasquelle kann ein Beatmungsgasvorrat, eine Anschlussformation zum Anschluss an eine gebäudeseitige Installation eines Beatmungsgasvorrats, wie es in Kliniken häufig der Fall ist, oder schlicht die Umgebungsluft in Kombination mit einem die Umgebungsluft in die Beatmungsleitungsanordnung fördernden Gebläse sein.

Die vorliegende Erfindung wird nachfolgend anhand der beiliegenden Zeichnungen näher erläutert werden. Es stellt dar:
- Fig.1: eine schematische Explosionsdarstellung einer erfindungsgemäßen Beatmungsvorrichtung,
- Fig.2: eine grobschematische Querschnittsdarstellung durch die Messküvette von Figur 1 mit der daran aufgenommenen erfindungsgemäßen Mehrkanal-Strahlungssensorbaugruppe von Figur 1 im Längsschnitt,
- Fig.3: eine Draufsicht auf die Strahlenteileranordnung, die vier ihre jeweils zugeordneten Messstrahlung-Sensoren verdeckenden Bandfilter von Figur 2 bei Betrachtung längs der Einstrahlachse ausgehend von der Schnittebene III-III in Figur 2,
- Fig. 4: eine grobschematische erste Querschnittsansicht einer möglichen refraktiv wirkenden Strahlenteileranordnung, und
- Fig. 5: eine grobschematische zweite Querschnittsansicht eine seines bevorzugten refraktiv wirkenden Strahlenteileranordnung mit konvex gekrümmter Oberflächen-Einhüllenden.

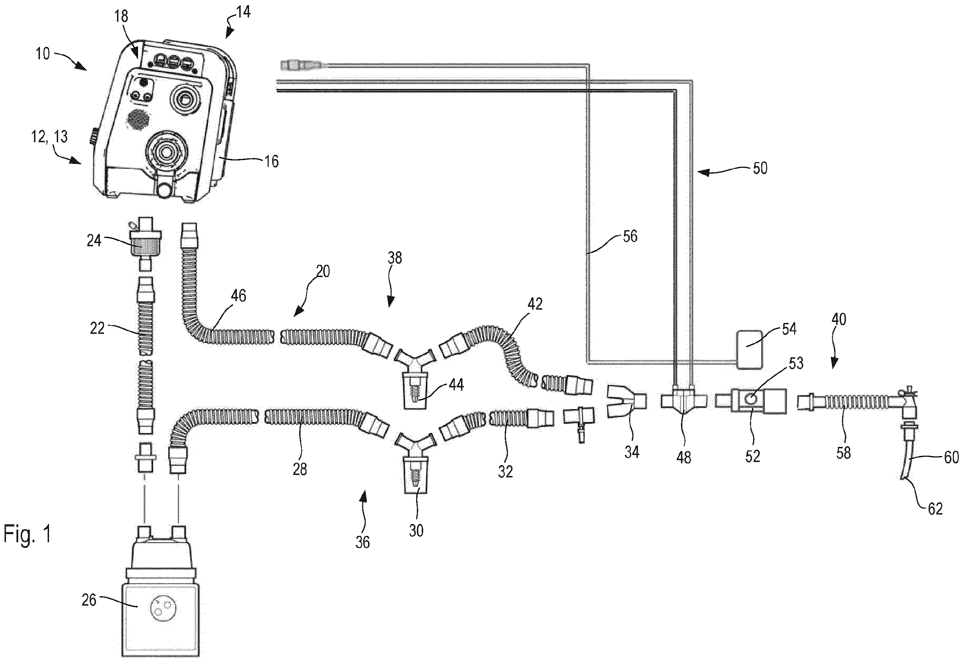

In Figur 1 ist eine erfindungsgemäße Ausführungsform einer Beatmungsvorrichtung allgemein mit 10 bezeichnet. Die Beatmungsvorrichtung 10 umfasst eine Beatmungsgasquelle 12 in Gestalt eines Gebläses sowie eine Steuervorrichtung 14 zur Einstellung von Betriebsparametern der Beatmungsgasquelle 12. Die Beatmungsgasquelle 12 und die Steuervorrichtung 14 sind im selben Gehäuse 16 aufgenommen. In diesem Gehäuse befinden sich auch an sich bekannte Ventile, wie ein Inspirationsventil und ein Exspirationsventil. Diese sind in Figur 1 jedoch nicht eigens dargestellt.

Die Steuervorrichtung 14 der Beatmungsvorrichtung 10 weist eine Eingabe/Ausgabeeinrichtung 18 auf, welche zahlreiche Schalter, wie Tastschalter und Drehschalter umfasst, um erforderlichenfalls Daten in die Steuervorrichtung 14 eingeben zu können. Das Gebläse der Beatmungsgasquelle 12 kann in seiner Förderleistung durch die Steuervorrichtung verändert werden, um die Menge an Beatmungsgas, das von der Beatmungsgasquelle pro Zeiteinheit gefördert wird, zu verändern. Die Beatmungsgasquelle 12 ist daher im vorliegenden Ausführungsbeispiel auch eine Druckveränderungsvorrichtung 13 der Beatmungsvorrichtung.

An die Beatmungsgasquelle 12 ist eine Beatmungsleitungsanordnung 20 angeschlossen, welche im vorliegenden Beispiel fünf flexible Schläuche umfasst. Ein erster inspiratorischer Beatmungsschlauch 22 verläuft von einem zwischen der Beatmungsgasquelle 12 und ihm selbst angeordneten Filter 24 zu einer Konditionierungsvorrichtung 26, wo das von der Beatmungsgasquelle 12 gelieferte Beatmungsgas auf einen vorgegebenen Feuchtegrad befeuchtet und gegebenenfalls mit Aerosol-Medikamenten versehen wird. Der Filter 24 filtert und reinigt die vom Gebläse als der Beatmungsgasquelle 12 gelieferte Umgebungsluft.

Ein zweiter inspiratorischer Beatmungsschlauch 28 führt von der Konditionierungsvorrichtung 26 zu einer inspiratorischen Wasserfalle 30. Ein dritter inspiratorischer Beatmungsschlauch 32 führt von der Wasserfalle 30 zu einem Y-Verbinder 34, welcher die distale Inspirationsleitung 36 und die distale Exspirationsleitung 38 zu einer kombinierten proximalen inspiratorisch-exspiratorischen Beatmungsleitung 40 verbindet.

Vom Y-Verbinder 34 zurück zum Gehäuse 16 verläuft ein erster exspiratorischer Beatmungsschlauch 42 zu einer exspiratorischen Wasserfalle 44 und von dort ein zweiter exspiratorischer Beatmungsschlauch 46 zum Gehäuse 16, wo das exspiratorische Beatmungsgas über ein nicht dargestelltes Exspirationsventil in die Umgebung abgelassen wird.

Auf der patientennahen kombinierten inspiratorisch-exspiratorischen Seite des Y-Verbinders 34 folgt unmittelbar auf den Y-Verbinder 34 ein Durchflusssensor 48, hier: ein Differenzdruck-Durchflusssensor 48, welcher den inspiratorischen und exspiratorischen Fluss an Beatmungsgas zum Patienten hin und vom Patienten weg erfasst. Eine Leitungsanordnung 50 überträgt den beiderseits eines Strömungshindernisses im Durchflusssensor 48 herrschenden Gasdruck an die Steuervorrichtung 14, die aus den übertragenen Gasdrücken und insbesondere aus der Differenz der Gasdrücke die Menge an pro Zeiteinheit strömendem inspiratorischem und exspiratorischem Beatmungsgas errechnet.

In Richtung vom Y-Verbinder 34 weg folgt zum Patienten hin auf den Durchflusssensor 48 eine Messküvette 52 zur nichtdispersiven Infrarot-Erfassung eines vorbestimmten Gasanteils im Beatmungsgas. Das Beatmungsgas ist folglich im vorliegenden Beispiel das in der Beschreibungseinleitung genannte Messfluid. Infrarotstrahlung ist im vorliegenden Beispiel die in der Beschreibungseinleitung genannte Messstrahlung. Der zu erfassende Messfluidbestandteil ist vorliegend CO₂. Ermittelt werden soll der Anteil an CO₂ im Beatmungsgas. Dabei ist sowohl der CO₂-Anteil bevorzugt sowohl im inspiratorischen Beatmungsgas wie auch im exspiratorischen Beatmungsgas von Interesse, da die Änderung des CO₂-Anteils zwischen Inspiration und Exspiration ein Maß für die Stoffwechselfähigkeit der Patientenlunge ist. Zu erkennen ist in Figur 1 eines der seitlichen Fenster 53, durch welche Infrarotlicht in die Messküvette 52 eingestrahlt werden kann bzw. aus dieser ausstrahlen kann, je nach Orientierung einer mit der Messküvette lösbar gekoppelten Mehrkanal-Strahlungssensorbaugruppe 54.

Die Sensorbaugruppe 54 ist an die Messküvette 52 derart ankoppelbar, dass die Sensorbaugruppe 54 die Messküvette 52 mit Infrarotlicht durchleuchten kann. Aus der Intensität des Infrarotlichts, genauer aus dessen spektraler Intensität kann in an sich bekannter Weise auf die Menge bzw. den Anteil eines vorbestimmten Gases in dem die Messküvette 52 durchströmenden Messfluid bzw. Messgas geschlossen werden. Der vorbestimmte Gasbestandteil, hier: CO₂, absorbiert Infrarotlicht einer definierten Wellenlänge. Die Intensität des Infrarotlichts in dieser Wellenlänge hängt nach Durchgang durch die Messküvette 52 im Wesentlichen von der Absorption des Infrarotlichts dieser Wellenlänge durch den vorbestimmten Gasbestandteil ab. Ein Vergleich der Intensität des Infrarotlichts der definierten Wellenlänge mit einer Wellenlänge des Infrarotlichts, die zu keinem Absorptionsspektrum eines zu erwartenden Gasanteils im Messgas gehört, liefert eine Information über den Anteil des vorbestimmten Gasbestandteils am Messgas. Die Sensorbaugruppe 54 ist daher über eine Datenleitung 56 mit der Steuervorrichtung 14 der Beatmungsvorrichtung 10 verbunden und überträgt über die Datenleitung 56 die beschriebenen Intensitätsinformationen an die Steuervorrichtung 14.

Auf die Messküvette 52 folgt in Richtung zum Patienten hin ein weiteres Schlauchstück 58, an welchen ein Endotrachealtubus 60 als Beatmungsschnittstelle zum Patienten angeordnet ist. Eine proximale Öffnung 62 des Endotrachealtubus 60 ist sowohl Beatmungsgas-Auslassöffnung, durch welche inspiratorisches Beatmungsgas durch den Endotrachealtubus 60 in den Patienten eingeleitet wird, als auch Beatmungsgas-Einlassöffnung, durch welche exspiratorisches Beatmungsgas aus dem Patienten zurück in den Endotrachealtubus 60 geleitet wird.

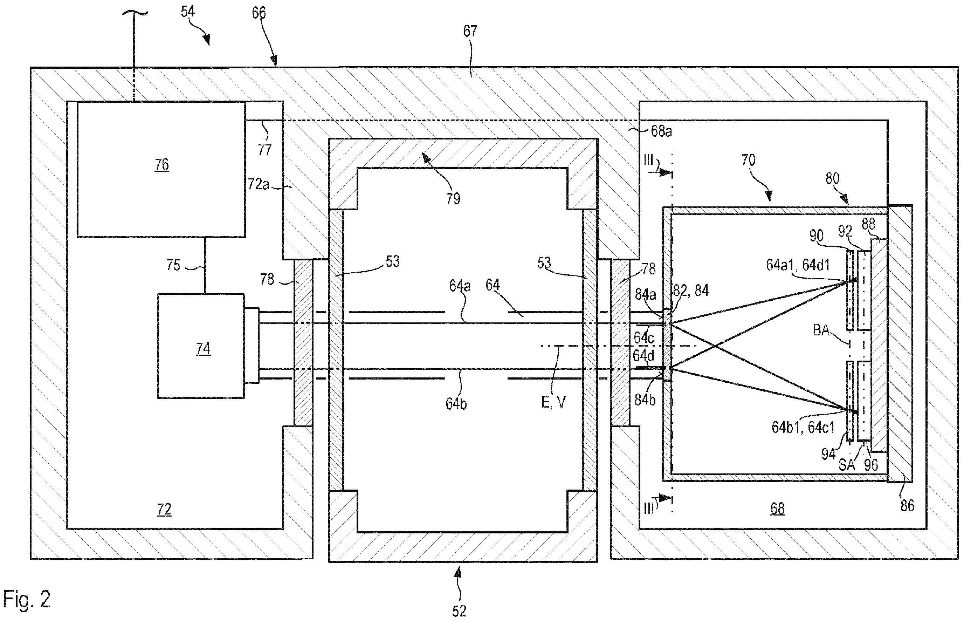

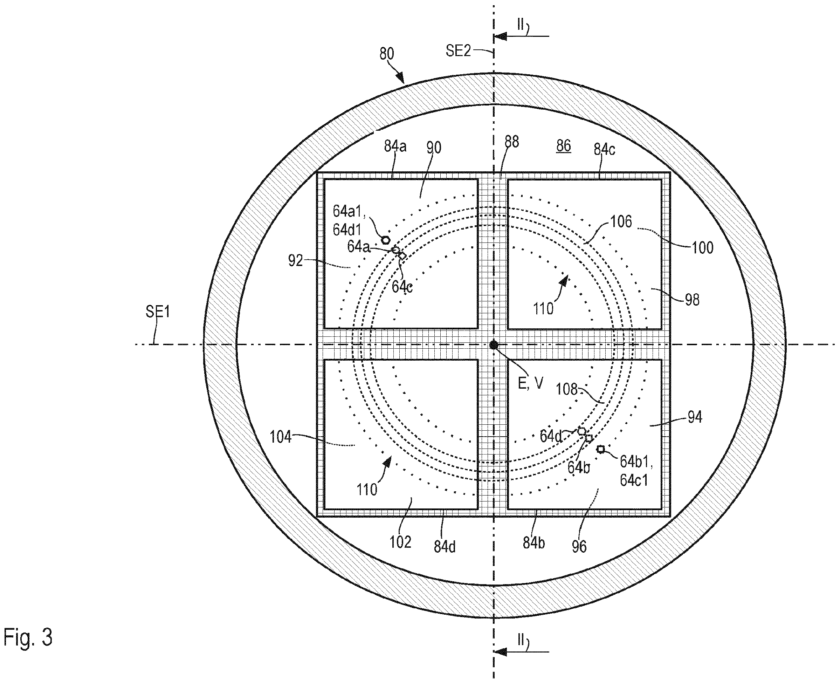

In Figur 2 sind die Messküvette 52 grobschematisch im Querschnitt und die darin angekoppelte Mehrkanal-Strahlungssensorbaugruppe 54 grobschematisch im Längsschnitt dargestellt. Die Messküvette 52 in Figur 2 wird orthogonal zur Zeichenebene von Figur 2 mit Beatmungsgas durchströmt. Ein Infrarotstrahl 64 der Sensorbaugruppe 54 verläuft parallel zur bzw. in der Zeichenebene von Figur 2. Die Zeichenebene der Figur 2 entspricht mit Ausnahme der Lage der Strahlengänge der Teilstrahlen 64a, 64b, 64c und 64d der Ebene II-II in Figur 3.

Die Sensorbaugruppe 54 umfasst ein Sensorgehäuse 66, in dessen erstem Kompartiment 68 eine weiter unten näher erläuterte Sensorik 70 angeordnet ist, und umfasst ein zweites Kompartiment 72, in welchem eine Infrarot-Strahlenquelle 74 Als Messstrahlungsquelle angeordnet ist. Lediglich beispielhaft ist im zweiten Kompartiment 72 eine bordeigene Sensor-Steuervorrichtung 76 angeordnet, welche über Leitungen 75 und 77 mit der Infrarot-Strahlenquelle 74 und der Sensorik 70 signalübertragend kommuniziert und welche über die Datenleitung 56 signalübertragend mit der Steuervorrichtung 14 kommuniziert. Im vorliegenden Beispiel kann die Steuervorrichtung 14 der Beatmungsvorrichtung 10 als übergeordnete Steuervorrichtung der Sensorbaugruppe 54 agieren und Erfassungswerte von der Sensor-Steuervorrichtung 76 anfordern, die daraufhin die Infrarot-Strahlenquelle 74 entsprechend zum Betrieb ansteuert und die von der Sensorik 70 erfassten Erfassungssignale an die Steuervorrichtung 14 zur Auswertung durch diese übertragt. Die Steuervorrichtung 14 ist somit eine Auswertevorrichtung der Sensorbaugruppe 54.

Die beiden Kompartimente 68 und 72 sind durch eine Gehäusebrücke 67 überbrückt. Die Gehäusebrücke 67 und die daran anschließenden Seitenwände 68a und 72a der beiden Kompartimente 68 und 72 bilden eine Klemm-Aufnahmeformation 79, in welche die Messküvette 52 eingeführt und lösbar klemmend verankert werden kann. Durch bloßes manuelles Überwinden der Klemmkraft können die Messküvette 52 und das Gehäuse 66 der Sensorbaugruppe 54 wieder voneinander getrennt werden. Zusätzlich oder alternativ können Verrastungsmittel zur Verrastung von Sensorbaugruppe 54 und Messküvette 52 aneinander vorgesehen sein.

Jedes der Kompartiment der 68 und 72 weist je ein infrarotdurchlässiges Fenster 78 auf, welches von dem von der Infrarot-Strahlenquelle 74 ausgestrahlten Infrarotstrahl 64 durchstrahlt wird. Da der Infrarotstrahl 64 die Messküvette 52 vollständig durchqueren muss, weist die Messküvette 52 beiderseits des durch sie bestimmten Strömungsdurchgangs je ein Fenster 53 auf, welches für Infrarotlicht durchlässig ist und welches von dem Infrarotstrahl 64 ebenso durchquert wird. Die Messküvette 52 ist in dem in der Sensorbaugruppe 54 aufgenommenen Abschnitt bevorzugt spiegelsymmetrisch bezüglich einer zum Infrarotstrahl 64 orthogonalen Spiegelsymmetrieebene ausgebildet, da es auf die Richtung der Durchstrahlung der Messküvette 52 durch den Infrarotstrahl 64 nicht ankommt. Die Sensorbaugruppe 54 kann daher abweichend von der Darstellung der Figur 2 auch um 180° um eine zur Zeichenebene der Figur 2 und zum Infrarotstrahl 64 orthogonale Achse gedreht mit der Messküvette 52 gekoppelt sein.

Die Sensorik 70 weist ein eigenes Sensorik-Gehäuse 80 auf. Das Sensorik-Gehäuse 80 umfasst ein Fenster 82, durch welches der Infrarotstrahl 64 längs einer Einstrahlachse E in das Sensorik-Gehäuse 80 einstrahlen kann.

Das Fenster 82 ist im dargestellten Ausführungsbeispiel eine Strahlenteileranordnung 84, welche in unterschiedlichen Einstrahlbereichen einstrahlendes Infrarotlicht in unterschiedliche Richtungen ablenkt. Dies wird weiter unten ausführlich erläutert. Alternativ zur Ausbildung des Fensters 82 als Strahlenteileranordnung 84 kann das Fenster 82 als lediglich durchlässiges optisch unwirksames Fenster ausgebildet sein. Es kann dann eine gesondert vom Fenster 82 ausgebildete Strahlenteileranordnung 84 hinter dem Fenster 82 im Sensorik-Gehäuse 80 angeordnet sein.

Das Sensorik-Gehäuse 80 weist eine Rückwand 86 auf, auf welchem ein Substrat 88 angeordnet ist, das eine Mehrzahl von Messstrahlung- bzw. Infrarot-Sensoren 92, 96, 100 und 104 trägt (siehe auch Figur 3). Das Substrat 88 kann mit signalübertragenden Leitungen ausgebildet sein, um Signale zwischen den Infrarot-Sensoren 92, 96, 100 und 104 und der Sensor-Steuervorrichtung 76 zu übertragen.

Vor jedem Infrarot-Sensor befindet sich je ein Bandfilter. Genauer befindet sich ein erster Bandfilter 90 vor dem Infrarot-Sensor 92, ein zweiter Bandfilter 94 vor dem Infrarot-Sensor 96, ein dritter Bandfilter 98 vor dem Infrarot-Sensor 100 und ein vierter Bandfilter 102 vor dem Infrarot-Sensor 104.

Der erste und der dritte Bandfilter 90 bzw. 98 weisen ein Transmissionsmaximum im Bereich der Absorptionswellenlängen von CO₂ auf, etwa in einem Bereich zwischen 4,25 µm und 4,28 µm. Ihre jeweiligen Bandbreiten liegen im Bereich von 170 bis 180 nm.

Der zweite und der vierte Bandfilter 94 bzw. 102 weisen ein Transmissionsmaximum im Bereich außerhalb der Absorptionswellenlängen von CO₂ auf, etwa im Bereich von 3,90 µm bis 4,0 µm, oder/und im Bereich von 4,40 µm bis 4,5 µm. Ihre jeweiligen Bandbreiten liegen im Bereich von 60 bis 90 nm.

Somit ist der Infrarot-Sensor 92 aufgrund der dargelegten Filterwerte des ersten Bandfilters 90 ein erster Messstrahlung- bzw. Infrarot-Nutzsignal-Sensor 92 und ist der Infrarotsensor 96 ein erster Messstrahlung- bzw. Infrarot-Referenzsignal-Sensor 96. Ebenso ist aufgrund der Filterwerte des dritten Bandfilters 98 der Infrarot-Sensor 100 ein zweiter Messstrahlung- bzw. Infrarot-Nutzsignal-Sensor 100. Schließlich ist der Infrarot-Sensor 104 ein zweiter Messstrahlung- bzw. Infrarot-Referenzsignal-Sensor 104. Bei entsprechender Wahl von Transmissionsmaximum und Bandbreite des vierten Bandfilters 102 kann der der Infrarot-Sensor 104 alternativ ein dritter Messstrahlung- bzw. Infrarot-Referenzsignal-Sensor 104 sein.

Der Infrarotstrahl 64 ist in Figur 2 in seinen äußersten Abmessungen lediglich mit unterbrochener Linie dargestellt. Mit durchgezogene Linie sind zwei im Folgenden näher betrachtete Teilstrahlen 64a und 64b des Infrarotstrahls 64 dargestellt. Zusätzlich sind zwei nahe bei den Teilstrahlen 64a und 64b des Infrarotstrahls 64 gelegene Nachbar-Teilstrahlen 64c und 64d dargestellt. Wie in Figur 3 gezeigt ist und noch näher erläutert werden wird, verlaufen die Teilstrahlen 64a bis 64d nicht in der Zeichenebene der Figur 2, sondern zum Teil davor, so die Teilstrahlen 64b und 64d, und zum Teil dahinter, so die Teilstrahlen 64a und 64c. Die Teilstrahlen sollen dabei als lokale Strahlenbereiche des Infrarotstrahls 64 verstanden werden.

Der Infrarotstrahl 64 strahlt als Messstrahlung kollimiertes Infrarotlicht längs der Einstrahlachse E, die auch optische Achse V der Strahlenteileranordnung 84 ist, auf die Strahlenteileranordnung 84 ein. Die Teilstrahlen 64a und 64c strahlen dabei in einem ersten Einstrahlbereich 84a auf die Strahlenteileranordnung 84 ein und die Teilstrahlen 64b und 64d strahlen in einem zweiten Einstrahlbereich 84b auf die Strahlenteileranordnung 84 ein. In Figur 3 sind die ersten und zweiten Einstrahlbereiche 84a und 84b sowie ein dritter Einstrahlbereich 84c und ein vierter Einstrahlbereich 84d dargestellt, welche bei der Betrachtung der Strahlenteileranordnung 84 längs der Einstrahlachse E von Figur 3 mit der Projektion der Flächen der Bandfilter 90, 94, 98 und 102 längs der Einstrahlachse E auf die Strahlenteileranordnung 84 zusammenfallen.

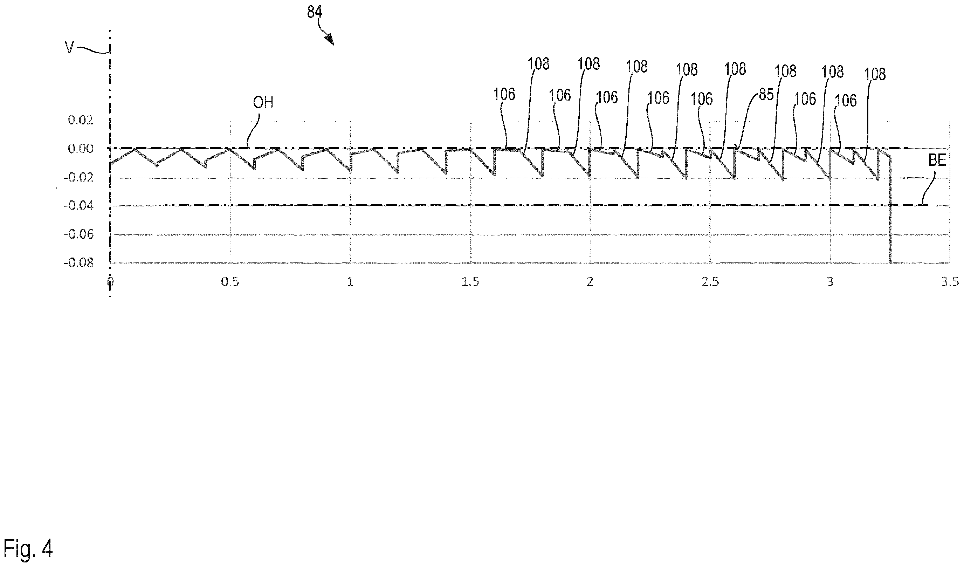

Die Strahlenteileranordnung 84 kann als refraktive Strahlenteileranordnung 84 eine Mehrzahl von in konzentrischen Ringen um die optische Achse V herum angeordnete erste Ablenkzonen 106 und zweite Ablenkzonen 108 (siehe auch Figur 4) aufweisen, die auf sie einstrahlende Infrarotstrahlung in unterschiedliche Richtungen ablenken.

Wie in Figur 2 und insbesondere in Figur 3 dargestellt ist, trifft der Teilstrahl 64a im ersten Einstrahlbereich 84a in einer ersten Ablenkzone 106 auf die Strahlenteileranordnung 84 und wird austrittsseitig auf einen Auftreffort 64a1 auf dem ersten Bandfilter 90 abgelenkt. Der Teilstrahl 64b trifft im zweiten Einstrahlbereich 84b in der ersten Ablenkzone 106 auf die Strahlenteileranordnung 84 und wird austrittseitig auf einen Auftreffort 64b1 auf dem zweiten Bandfilter 94 abgelenkt. Die erste Ablenkzone 106 läuft vollständig und kontinuierlich um die optische Achse V um und ist somit eine einheitliche erste Ablenkzone 106 für beide Einstrahlbereiche 84a und 84b. Sie lenkt einen parallel zur optischen Achse V einstrahlenden Teilstrahl in einer die optische Achse V enthaltenden virtuellen Ebene von seiner ursprünglich zur optischen Achse parallelen Bahn ab.

Der Teilstrahl 64c trifft in dem ersten Einstrahlbereich 84a in der zweiten Ablenkzone 108 auf die Strahlenteileranordnung 84 und wird zu einem Auftreffort 64c1 auf dem zweiten Bandfilter 94 abgelenkt. Der Teilstrahl 64d trifft im zweiten Einstrahlbereich 84b in derselben zweiten Ablenkzone 108 auf die Strahlenteileranordnung 84 und wird auf einen Auftreffort 64d1 auf dem ersten Bandfilter 90 abgelenkt. Auch die zweite Ablenkzone 108 lenkt einen parallel zur optischen Achse V einstrahlenden Teilstrahl in einer die optische Achse V enthaltenden virtuellen Ebene von seiner ursprünglich zur optischen Achse parallelen Bahn ab. Allerdings erfolgt diese Ablenkung mit einem anderen Ablenkwinkel als in der ersten Ablenkzone 106. Vorzugsweise schneiden die von der zweiten Ablenkzone 108 abgelenkten Teilstrahlen im Gegensatz zu den in derselben Ebene liegenden und von der ersten Ablenkzone abgelenkten Teilstrahlen die optische Achse V.

Die Auftrefforte 64a1 und 64d1 auf dem ersten Bandfilter 90 sind vorzugsweise dieselben Auftrefforte oder liegen nahe bei einander. Gleiches gilt für die Auftrefforte 64b1 und 64c1 auf dem zweiten Bandfilter 94.

Von den mehreren ersten Ablenkzonen 106 ist in Figur 3 der besseren Übersichtlichkeit halber nur eine einzige gezeigt; ebenso ist nur eine zweite Ablenkzone 108 von mehreren zweiten Ablenkzonen 108 in Figur 3 dargestellt. Längs einer beliebigen orthogonalen Linie, ausgehend von der optischen Achse V der Strahlenteileranordnung 84, sind alternierend erste und zweite Ablenkzonen 106 bzw. 108 aufeinanderfolgend angeordnet oder ausgebildet.

Insgesamt wird der auf die Strahlenteileranordnung 84 mit einem Kreisquerschnitt auftreffende Infrarotstrahl 64 durch die Strahlenteileranordnung 84 derart geteilt und abgelenkt, dass ein Intensitätsmaximum des den ersten bis vierten Bandfilter 90, 94, 98 und 102 erreichenden Infrarotlichts in einem locker punktiert dargestellten Ringbereich 110 gelegen ist. Die Bandfilter 90, 94, 98 und 102 sind somit im Wesentlichen gleichmäßig ausgeleuchtet. Aufgrund der rotationssymmetrischen Ausbildung der Strahlenteileranordnung 84, zumindest von deren refraktiv wirksamer Grenzfläche 85, bezüglich der optischen Achse V ist bei symmetrisch bezüglich der optischen Achse V ebenfalls symmetrisch einstrahlender Infrarotstrahlung 64 auch die im Bereich der Bandfilter 90, 94, 98 und 102 und in der Folge auch im Bereich der Messstrahlung-Sensoren 92, 96, 100 und 104 durch die Strahlenteileranordnung 84 erzeugte Abbildung rotationssymmetrisch.

Die vorzugsweise eben ausgebildeten Bandfilter 90, 94, 98 und 102 liegen in einer gemeinsamen zur optischen Achse V orthogonalen Bandfilter-Anordnungsebene BA. Ebenso liegen die vorzugsweise ebenen Sensorflächen aufweisenden Messstrahlung-Sensoren 92, 96, 100 und 104 in einer gemeinsamen zur optischen Achse V orthogonalen sowie zur Bandfilter-Anordnungsebene BA parallelen Sensor-Anordnungsebene SA. So kann das Sensorik-Gehäuse 80 und somit im Wesentlichen die Sensorbaugruppe 10 insgesamt längs der optischen Achse V mit geringer Abmessung realisiert werden.

Wie in Figur 3 gut zu erkennen ist, sind die Bandfilter 90, 94, 98 und 102 in ihrer Bandfilter-Anordnungsebene BA als rechteckige Bandfilter unter guter Bauraumausnutzung gekachelt angeordnet. Die Anordnung der in Figur 3 hinter den Bandfiltern 90, 94, 98 und 102 gelegenen Messstrahlung-Sensoren 92, 96, 100 und 104 entspricht exakt jener der Bandfilter 90, 94, 98 und 102. Einander in der jeweiligen Anordnungsebene BA bzw. SA benachbarte Bauteile: Bandfilter und Messstrahlung-Sensoren, liegen in der jeweiligen Anordnungsebene mit zueinander parallelen Rändern nebeneinander. Durch die Kachelung mit rechteckigen Bauteilen sind sowohl die Bandfilter 90, 94, 98 und 102 als auch die Messstrahlung-Sensoren 92, 96, 100 und 104 zu zueinander orthogonalen Symmetrieebenen SE1 und SE2 jeweils spiegelsymmetrisch angeordnet. Die Symmetrieebene SE1 und SE2 sind orthogonal zur Zeichenebene der Figur 3. Bevorzugt ist die Schnittgerade der Symmetrieebenen SE1 und SE2 die optische Achse V. Im dargestellten Ausführungsbeispiel ist außerdem die Symmetrieebene SE2 die Schnittebene der Darstellung von Figur 2.

In Figur 4 ist ein Profilschnitt durch eine mögliche Ausführungsform der Strahlenteileranordnung 84 in einer die optische Achse V enthaltenden Schnittebene dargestellt. Der Schnitt durch die refraktiv wirksame Grenzfläche 85 der Strahlenteileranordnung 84 zeigt die Lage der in Richtung von der optischen Achse V weg alternierend aufeinander folgenden ersten Ablenkzonen 106 und zweiten Ablenkzonen 108. Die ersten Ablenkzonen 106 weisen einen Grenzflächenabschnitt auf, welcher bezüglich einer zur optischen Achse V orthogonalen Bezugsebene BE weniger stark geneigt ist. Dagegen weisen die zweiten Ablenkzonen 108 einen Grenzflächenabschnitt auf, welcher bezüglich der zur optischen Achse V orthogonalen Bezugsebene BE stärker geneigt ist. Abhängig von ihrem Abstand von der optischen Achse V ändert sich die Neigung der ersten und der zweiten Ablenkzonen 106 bzw. 108 bezüglich der Bezugsebene. Aufgrund der gewählten Anordnung der Strahlenteileranordnung 84 einerseits und der Bandfilter 90, 94, 98 und 102 sowie Messstrahlung-Sensoren 92, 96, 100 und 104 andererseits ändert sich mit zunehmender Entfernung von der optischen Achse V sogar der Neigungssinn der ersten Ablenkzonen 106, also das Vorzeichen des Winkels, mit welchem die ersten Ablenkzonen 106 bezüglich der Bezugsebene BE geneigt sind. Die Neigung der ersten Ablenkzonen 106 wird daher mit zunehmenden Abstand von der optischen Achse V größer, da zunächst ein negativer Neigungswinkel im Profilschnitt besteht, welcher abnimmt bis sich das Vorzeichen des Neigungswinkel ändert und der Neigungswinkel mit positiven Vorzeichen dann betragsmäßig mit zunehmenden Abstand von der optischen Achse V größer wird. Auch die Neigung der zweiten Ablenkzonen 108 nimmt mit größer werdendem Abstand von der optischen Achse V zu.

Der besseren Übersichtlichkeit halber sind in Figur 4 nicht alle ersten Ablenkzonen 106 und nicht alle zweiten Ablenkzonen 108 mit einem Bezugszeichen versehen.

Die Ausführungsform der Strahlenteileranordnung 84 von Figur 4 weist eine ebene und zur optischen Achse V orthogonale Oberflächen-Einhüllende OH auf.

Grundsätzlich kann eine Strahlenteileranordnung 84 auch mit ebener Oberflächen-Einhüllender OH an der Strahlungssensorbaugruppe 54 eingesetzt werden. Jedoch kann es dabei vorkommen, dass die Fläche des Ringbereichs 110, in welche die kollimiert einstrahlende Infrarotstrahlung 64 von der Strahlenteileranordnung 84 abgelenkt wird, nicht optimal auf die Oberflächen der Bandfilter 90, 94, 98 und 102 einstrahlt und somit die Sensorbaugruppe 10 zwar funktioniert, jedoch hinsichtlich des erreichbaren Signalpegels noch verbessert werden kann.

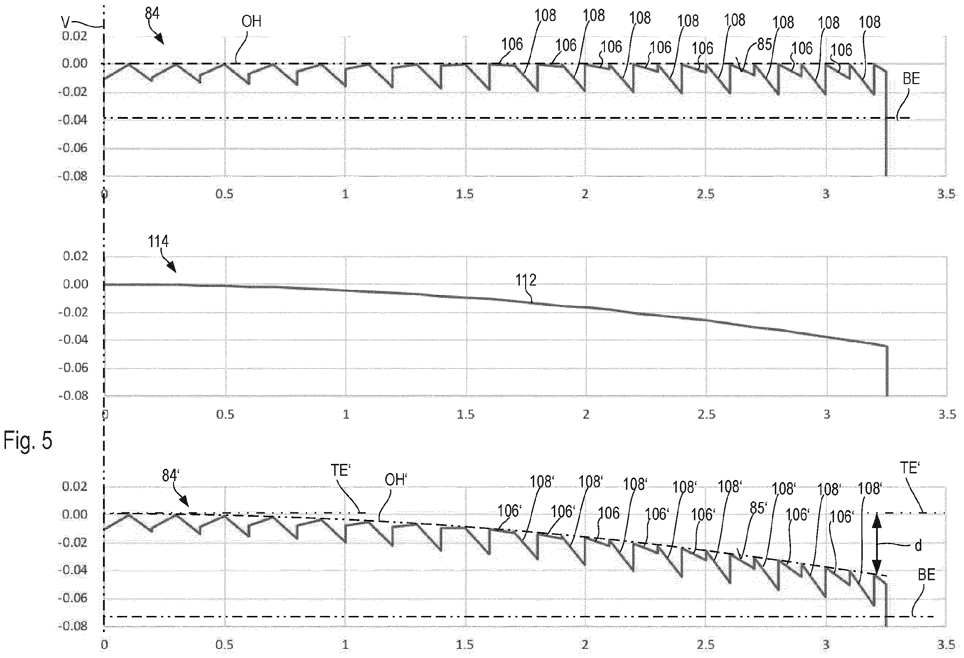

Figur 5 zeigt eine optimierte Gestalt der refraktiv wirksamen Grenzfläche 85' einer besonders bevorzugten modifizierten Strahlenteileranordnung 84'. Sie ist gebildet durch eine Überlagerung der Grenzfläche 85 der Strahlenteileranordnung 84 von Figur 4 mit einer konvex gekrümmten Grundfläche 112 eines optischen Rohlings 114 zur Herstellung einer Strahlenteileranordnung 84. Das Ergebnis dieser Superposition ist die in Figur 5, unten, gezeigte refraktiv wirksame Grenzfläche 85' der modifizierten Strahlenteileranordnung 84'. Da dort die immer noch in konzentrischen Ringen um die optische Achse V der Strahlenteileranordnung 84 'ausgebildeten ersten Ablenkzonen 106' und zweiten Ablenkzonen 108' auf einer konvex gekrümmten Grundfläche 112 ausgebildet sind, ist die refraktiv wirksame Grenzfläche 85' nicht mehr durch eine ebene Oberflächen-Einhüllende OH, sondern durch eine konvex gekrümmte Oberflächen-Einhüllende OH' virtuell eingefasst. Durch entsprechende Einstellung der konvexen Krümmung der Grundfläche 112 kann der Radius der Ringfläche 110 mit der größten Intensität der austrittseitigen Abbildung der Strahlenteileranordnung 84 bzw. 84' eingestellt werden. Somit kann die Abbildungs-Ringfläche 110 durch geeignete Wahl der konvexen Krümmung der Grundfläche 112 bzw. der Oberflächen-Einhüllenden OH' auf die jeweilige Lage der Bandfilter 90, 94, 98 und 102 sowie der Messstrahlung-Sensoren 92, 96, 100 und 104 optimal angepasst werden.

Die konvexe Oberflächen-Einhüllende OH' krümmt sich in jeder zur optischen Achse V orthogonalen Richtung von einer zur optischen Achse V orthogonalen Tangentialebene TE' an die refraktiv wirksame Grenzfläche 85' der Strahlenteileranordnung 84' derart weg, dass ein parallel zur optischen Achse V zu messender Abstand d zwischen der Oberflächen-Einhüllenden OH' und der Tangentialebene TE' mit zunehmendem Abstand von der optischen Achse V größer wird. Zur Vermeidung unnötigen Materialaufwands liegt vorzugsweise die gesamte Strahlenteileranordnung 84', wenigstens jedoch deren refraktiv wirksame Grenzfläche 85' vollständig auf ein und derselben Seite der Tangentialebene TE' und schneidet diese nicht.

## Patentansprüche

1. Nichtdispersive Mehrkanal-Strahlungssensorbaugruppe (54) zur quantitativen Ermittlung eines eine elektromagnetische Messstrahlung absorbierenden Bestandteils eines Messfluids, umfassend:
- eine Strahlenteileranordnung (84, 84'), welche dazu ausgebildet ist, einen auf die Strahlenteileranordnung (84, 84') längs einer vorbestimmten Einstrahlachse (E) einstrahlenden Strahl (64) der Messstrahlung zu teilen,
- einen von einem ersten Teil (64a, 64c) der Messstrahlung erreichbaren ersten Bandfilter (90) mit einer vorbestimmten ersten Bandbreite und mit einem Transmissionsmaximum bei einer vorbestimmten ersten Nutzsignal-Wellenlänge,
- einen im Strahlengang hinter dem ersten Bandfilter (90) angeordneten ersten Messstrahlung-Nutzsignal-Sensor (92), auf welchen den ersten Bandfilter (90) durchstrahlende Messstrahlung einstrahlt,
- einen räumlich vom ersten Bandfilter (90) entfernt angeordneten zweiten Bandfilter (94), welcher von einem vom ersten Teil (64a, 64c) verschiedenen zweiten Teil (64b, 64d) der Messstrahlung erreichbar ist, wobei der zweite Bandfilter (94) eine vorbestimmte zweite Bandbreite und ein Transmissionsmaximum bei einer vorbestimmten ersten Referenzsignal-Wellenlänge aufweist, wobei die erste Referenzsignal-Wellenlänge von der ersten Nutzsignal-Wellenlänge verschieden ist,
- einen im Strahlengang hinter dem zweiten Bandfilter (94) und räumlich vom ersten Messstrahlung-Nutzsignal-Sensor (92) entfernt angeordneten ersten Messstrahlung-Referenzsignal-Sensor (96), auf welchen den zweiten Bandfilter (94) durchstrahlende Messstrahlung einstrahlt,
**dadurch gekennzeichnet, dass** die Strahlenteileranordnung (84, 84') eine von der Messstrahlung durchstrahlte und die durchstrahlende Messstrahlung brechende Strahlenteileranordnung (84, 84') ist, welche einen ersten Einstrahlbereich (84a) und einen räumlich vom ersten verschiedenen zweiten Einstrahlbereich (84b) aufweist, in welchen Einstrahlbereichen (84a, 84b) Messstrahlung auf die Strahlenteileranordnung (84, 84') einstrahlt, wobei der erste (84a) und der zweite Einstrahlbereich (84b) optisch derart ausgebildet sind, dass
- die Strahlenteileranordnung (84, 84') im ersten Einstrahlbereich (84a)
+ einen ersten Teil (64a) der auf den ersten Einstrahlbereich (84a) einstrahlenden Messstrahlung auf den ersten Bandfilter (90) ablenkt, und
+ einen zweiten Teil (64c) der auf den ersten Einstrahlbereich (84a) einstrahlenden Messstrahlung auf den zweiten Bandfilter (94) ablenkt,
und dass
- die Strahlenteileranordnung (84, 84') im zweiten Einstrahlbereich (84b)
+ einen ersten Teil (64b) der auf den zweiten Einstrahlbereich (84b) einstrahlenden Messstrahlung auf den zweiten Bandfilter (94) ablenkt und
+ einen zweiten Teil (64d) der auf den zweiten Einstrahlbereich (84b) einstrahlenden Messstrahlung auf den ersten Bandfilter (90) ablenkt,
wobei der erste Einstrahlbereich (84a) optisch refraktiv wirksam ist und wenigstens zwei die einstrahlende Messstrahlung jeweils in unterschiedliche Richtungen ablenkende Ablenkzonen (106, 108; 106', 108') aufweist, wobei eine erste Ablenkzone (106; 106') die Ablenkung des ersten Teils (64a) der auf den ersten Einstrahlbereich (84a) einstrahlenden Messstrahlung auf den ersten Bandfilter (90) bewirkt und wobei eine zweite Ablenkzone (108; 108') die Ablenkung des zweiten Teils (64c) der auf den ersten Einstrahlbereich (84a) einstrahlenden Messstrahlung auf den zweiten Bandfilter (94) bewirkt,
oder/und
der zweite Einstrahlbereich (84b) optisch refraktiv wirksam ist und wenigstens zwei die einstrahlende Messstrahlung jeweils in unterschiedliche Richtungen ablenkende Ablenkzonen (106, 108; 106', 108') aufweist, wobei eine erste Ablenkzone die Ablenkung des ersten Teils (64b) der auf den zweiten Einstrahlbereich (84b) einstrahlenden Messstrahlung bewirkt und wobei eine zweite Ablenkzone (108; 108') die Ablenkung des zweiten Teils (64d) der auf den zweiten Einstrahlbereich (84b) einstrahlenden Messstrahlung bewirkt, wobei die erste und die zweite Ablenkzone (106, 108; 106', 108') eines Einstrahlbereichs (84a, 84b, 84c, 84d) sich voneinander durch zu ihrer jeweiligen Ausbildung verwendete unterschiedliche Materialien mit unterschiedlichen Brechungsindizes oder/und durch lokal unterschiedliche Grenzflächengestalten an einer die Strahlenteileranordnung (84; 84') im Bereich ihres Einstrahlbereichs (84a, 84b, 84c, 84d) von ihrer Umgebung scheidenden Grenzfläche (85; 85') unterscheiden.

2. Strahlungssensorbaugruppe (54) nach Anspruch 1, **dadurch gekennzeichnet, dass** die erste und die zweite Ablenkzone (106, 108; 106', 108') auf ihren Einstrahlbereich (84a, 84b, 84c, 84d) einstrahlende Messstrahlung bezüglich einer optischen Achse (V) der Strahlenteileranordnung (84; 84') jeweils unterschiedlich stark ablenken.

3. Strahlungssensorbaugruppe (54) nach Anspruch 1 oder 2, **dadurch gekennzeichnet, dass** der erste Einstrahlbereich (84a) eine Mehrzahl von ersten oder/und von zweiten Ablenkzonen (106, 108; 106', 108') umfasst
oder/und
dass der zweite Einstrahlbereich (84b) eine Mehrzahl von ersten oder/und von zweiten Ablenkzonen (106, 108; 106', 108') umfasst.

4. Strahlungssensorbaugruppe (54) nach einem der Ansprüche 1 bis 3, **dadurch gekennzeichnet, dass** sich die erste und die zweite Ablenkzone (106', 108') eines Einstrahlbereichs (84a, 84b, 84c, 84d) voneinander durch lokal unterschiedliche Grenzflächengestalten an einer die Strahlenteileranordnung im Bereich ihres Einstrahlbereichs (84a, 84b, 84c, 84d) von ihrer Umgebung scheidenden Grenzfläche (85') unterscheiden, wobei ein die erste Ablenkzone (106') und die zweite Ablenkzone (108') aufweisender Grenzflächenbereich eine Oberflächen-Einhüllende (OH') aufweist, deren parallel zu einer optischen Achse (V) der Strahlenteileranordnung (84') zu messender Abstand (d) von einer zur optischen Achse (V) orthogonalen Tangentialebene (TE') an die Grenzfläche (85') mit zunehmender Entfernung von der optischen Achse (V) der Strahlenteileranordnung (84') größer wird.

5. Strahlungssensorbaugruppe (54) nach Anspruch 4, **dadurch gekennzeichnet, dass** ein refraktiv wirksamer Abschnitt der Strahlenteileranordnung (84; 84') spiegelsymmetrisch ausgebildet ist, insbesondere bezüglich einer die optische Achse (V) enthaltenden Symmetrieebene (SE1, SE2).

6. Strahlungssensorbaugruppe (54) nach einem der Ansprüche 2. 4 oder 5, **dadurch gekennzeichnet, dass** ein refraktiv wirksamer Abschnitt der Strahlenteileranordnung (84; 84') rotationssymmetrisch bezüglich der optischen Achse (V) als Rotationssymmetrieachse ausgebildet ist.

7. Strahlungssensorbaugruppe (54) nach einem der vorhergehenden Ansprüche, **dadurch gekennzeichnet, dass** wenigstens eine Ablenkzone der wenigstens einen ersten Ablenkzone (106; 106') des ersten (84a) und des zweiten Einstrahlbereichs (84b) wenigstens abschnittsweise, vorzugsweise vollständig, einstückig zusammenhängend ausgebildet ist oder/und wenigstens eine Ablenkzone der wenigstens einen zweiten Ablenkzone (108; 108') des ersten (84a) und des zweiten Einstrahlbereichs (84b) wenigstens abschnittsweise, vorzugsweise vollständig, einstückig zusammenhängend ausgebildet ist.

8. Strahlungssensorbaugruppe (54) nach einem der vorhergehenden Ansprüche, **dadurch gekennzeichnet, dass** die Strahlungssensorbaugruppe (54) weiter umfasst:
- einen räumlich vom ersten (90) und vom zweiten Bandfilter (94) entfernt angeordneten, von einem dritten Teil der Messstrahlung erreichbaren dritten Bandfilter (98) mit einer vorbestimmten dritten Bandbreite und mit einem Transmissionsmaximum bei einer vorbestimmten zweiten Nutzsignal-Wellenlänge,
- einen räumlich von dem ersten Messstrahlung-Nutzsignal-Sensor (92) und dem ersten Messstrahlung-Referenzsignal-Sensor (96) entfernt angeordneten, im Strahlengang hinter dem dritten Bandfilter (98) angeordneten zweiten Messstrahlung-Nutzsignal-Sensor (100), auf welchen den dritten Bandfilter (98) durchstrahlende Messstrahlung einstrahlt,
- einen räumlich vom ersten (90), zweiten (94) und dritten Bandfilter (98) entfernt angeordneten vierten Bandfilter (102), welcher von einem vom ersten, zweiten und dritten Teil verschiedenen vierten Teil der Messstrahlung erreichbar ist, wobei der vierte Bandfilter (102) eine vorbestimmte vierte Bandbreite und ein Transmissionsmaximum bei einer vorbestimmten zweiten Referenzsignal-Wellenlänge aufweist, wobei die zweite Referenzsignal-Wellenlänge von der zweiten Nutzsignal-Wellenlänge verschieden ist,
- einen im Strahlengang hinter dem vierten Bandfilter (102) und räumlich vom ersten (92) und zweiten Messstrahlung-Nutzsignal-Sensor (100) sowie vom ersten Messstrahlung-Referenzsignal-Sensor (96) entfernt angeordneten zweiten Messstrahlung-Referenzsignal-Sensor (104), auf welchen den vierten Bandfilter (102) durchstrahlende Messstrahlung einstrahlt,
wobei die Strahlenteileranordnung (84; 84') einen vom ersten (84a) und vom zweiten Einstrahlbereich (84b) räumlich verschiedenen dritten Einstrahlbereich (84c) sowie einen vom ersten (84a), zweiten (84b) und dritten Einstrahlbereich (84c) räumlich verschiedenen vierten Einstrahlbereich (84d) aufweist, wobei in den dritten und vierten Einstrahlbereichen (84c, 84d) Messstrahlung auf die Strahlenteileranordnung (84; 84') einstrahlt, und wobei der dritte (84c) und der vierte Einstrahlbereich (84d) optisch derart ausgebildet sind, dass
- die Strahlenteileranordnung (84; 84') im dritten Einstrahlbereich (84c)
+ einen ersten Teil der auf den dritten Einstrahlbereich (84c) einstrahlenden Messstrahlung auf den dritten Bandfilter (98) ablenkt, und
+ einen zweiten Teil der auf den dritten Einstrahlbereich (84c) einstrahlenden Messstrahlung auf den vierten Bandfilter (102) ablenkt,
und dass
- die Strahlenteileranordnung (84; 84') im vierten Einstrahlbereich (84d)
+ einen ersten Teil der auf den vierten Einstrahlbereich (84d) einstrahlenden Messstrahlung auf den vierten Bandfilter (102) ablenkt und
+ einen zweiten Teil der auf den vierten Einstrahlbereich (84d) einstrahlenden Messstrahlung auf den dritten Bandfilter (98) ablenkt.

9. Strahlungssensorbaugruppe (54) nach einem der vorhergehenden Ansprüche, **dadurch gekennzeichnet, dass** eine Mehrzahl von Bandfiltern (90, 94, 98, 102) oder/und eine Mehrzahl von Messstrahlung-Sensoren (92, 96, 100, 104) jeweils in einer Bandfilter-Anordnungsebene (BA) bzw. in einer Sensor- Anordnungsebene (SA) angeordnet sind.

10. Strahlungssensorbaugruppe (54) nach einem der vorhergehenden Ansprüche, **dadurch gekennzeichnet, dass** sie mit Abstand von der Strahlenteileranordnung (84; 84') eine Messstrahlungsquelle (74) aufweist, welche dazu ausgebildet ist, Messstrahlung zur Strahlenteileranordnung (84; 84') hin auszustrahlen.

11. Strahlungssensorbaugruppe (54) nach Anspruch 10, **dadurch gekennzeichnet, dass** die Messstrahlungsquelle (74) dazu ausgebildet ist, kollimierte Messstrahlung auszustrahlen.

12. Strahlungssensorbaugruppe (54) nach einem der vorhergehenden Ansprüche, **dadurch gekennzeichnet, dass** die Strahlungssensorbaugruppe (54) eine Auswertevorrichtung (14, 76) umfasst, welche aus den Signalen des ersten Messstrahlung-Referenzsignal-Sensors (96) eine Referenzinformation ermittelt, welche aus den Signalen des ersten Messstrahlung-Nutzsignal-Sensors (92) eine Nutzinformation ermittelt, und welche aus einem Vergleich von Referenzinformation und Nutzinformation eine Information über einen Anteil eines durch die erste Nutzsignal-Wellenlänge identifizierten Gasbestandteils an einem mit der Messstrahlung bestrahlten Messgas ausgibt.

13. Strahlungssensorbaugruppe (54) nach einem der vorhergehenden Ansprüche, unter Einbeziehung des Anspruchs 10, **dadurch gekennzeichnet, dass** die Strahlungssensorbaugruppe (54) ein Sensorgehäuse (67) aufweist mit einem ersten Kompartiment (68), an oder in welchem die Strahlenteileranordnung (84; 84'), der Messstrahlung-Nutzsignal-Sensor (92) und der Messstrahlung-Referenzsignal-Sensor (96) angeordnet sind, und mit einem räumlich vom ersten Kompartiment (68) entfernt gelegenen zweiten Kompartiment (72), an oder in welchem die Messstrahlungsquelle (74) angeordnet ist, wobei zwischen dem ersten (68) und dem zweiten Kompartiment (72) eine Aufnahmeformation (79) zur Aufnahme einer Messküvette (52) zwischen dem ersten (68) und dem zweiten Kompartiment (72) angeordnet ist.

14. Beatmungsvorrichtung (10) zur wenigstens unterstützend künstlichen Beatmung eines lebenden Patienten, umfassend:
- eine Beatmungsgasquelle (12),
- eine Beatmungsleitungsanordnung (20), um inspiratorisches Beatmungsgas von der Beatmungsgasquelle (12) zu einer patientenseitigen, proximalen Beatmungsgas-Auslassöffnung (62) und um exspiratorisches Beatmungsgas von einer proximalen Beatmungsgas-Einlassöffnung (62) weg zu leiten,
- eine Druckveränderungsvorrichtung (13) zur Veränderung des Drucks des Beatmungsgases in der Beatmungsleitungsanordnung (20),
- eine Steuervorrichtung (14) zum Betrieb der Beatmungsgasquelle (12) oder/und der Druckveränderungsvorrichtung (13) sowie
- eine Mehrkanal-Strahlungssensorbaugruppe (54) nach einem der vorhergehenden Ansprüche zur Erfassung wenigstens eines Gasbestandteils im inspiratorischen oder/und exspiratorischen Beatmungsgas.

## Claims

1. Non-dispersive multichannel radiation sensor assembly (54) for quantitative determination of an electromagnetic measuring radiation-absorbing component of a measuring fluid, comprising:
- a beam splitter arrangement (84, 84') configured to split a beam (64) of the measuring radiation incident on the beam splitter arrangement (84, 84') along a predetermined incidence axis (E),
- a first bandpass filter (90) reachable by a first part (64a, 64c) of the measuring radiation with a predetermined first bandwidth and with a transmission maximum at a predetermined first useful signal wavelength,
- a first measuring radiation useful signal sensor (92) arranged in the beam path behind the first bandpass filter (90) on which measuring radiation traversing the first bandpass filter (90) is incident,
- a second bandpass filter (94) arranged spatially distant from the first bandpass filter (90) which is reachable by a second part (64b, 64d) of the measuring radiation which is different from the first part (64a, 64c), wherein the second bandpass filter (94) exhibits a predetermined second bandwidth and a transmission maximum at a predetermined first reference signal wavelength, wherein the first reference signal wavelength is different from the first useful signal wavelength,
- a first measuring radiation reference signal sensor (96) arranged in the beam path behind the second bandpass filter (94) and spatially distant from the first measuring radiation useful signal sensor (92) on which measuring radiation traversing the second bandpass filter (94) is incident,
**characterized in that** the beam splitter arrangement (84, 84') is a beam splitter arrangement (84, 84') traversed by the measuring radiation and refracting the traversing measuring radiation, exhibiting a first incidence region (84a) and a second incidence region (84b) different spatially from the first, in which incidence regions (84a, 84b) measuring radiation is incident on the beam splitter arrangement (84, 84'), wherein the first (84a) and the second incidence region (84b) are configured optically in such a way that
- the beam splitter arrangement (84, 84') in the first incidence region (84a)
+ deflects a first part (64a) of the measuring radiation incident on the first incidence region (84a) onto the first bandpass filter (90), and
+ deflects a second part (64c) of the measuring radiation incident on the first incidence region (84a) onto the second bandpass filter (94),
and that
- the beam splitter arrangement (84, 84') in the second incidence region (84b)
+ deflects a first part (64b) of the measuring radiation incident on the second incidence region (84b) onto the second bandpass filter (94), and
+ deflects a second part (64d) of the measuring radiation incident on the second incidence region (84b) onto the first bandpass filter (90),
wherein the first incidence region (84a) is optically refractively active and exhibits at least two deflection zones (106, 108; 106', 108') which deflect the incident measuring radiation in different directions respectively, wherein a first deflection zone (106; 106') effects the deflection of the first part (64a) of the measuring radiation incident on the first incidence region (84a) onto the first bandpass filter (90) and wherein a second deflection zone (108; 108') effects the deflection of the second part (64c) of the measuring radiation incident on the first incidence region (84a) onto the second bandpass filter (94),
and/or
the second incidence region (84b) is optically refractively active and exhibits at least two deflection zones (106, 108; 106', 108') which deflect the incident measuring radiation in different directions respectively, wherein a first deflection zone effects the deflection of the first part (64b) of the measuring radiation incident on the second incidence region (84b) and wherein a second deflection zone (108; 108') effects the deflection of the second part (64d) of the measuring radiation incident on the second incidence region (84b),
wherein the first and the second deflection zone (106, 108; 106', 108') of an incidence region (84a, 84b, 84c, 84d) differ from one another through different materials with different refractive indices used in their respective configuration and/or through locally different interface shapes at an interface (85; 85') separating the beam splitter arrangement (84; 84') from its environment in the in the region of its incidence region (84a, 84b, 84c, 84d).

2. Radiation sensor assembly (54) according to Claim 1,
**characterized in that** the first and the second deflection zone (106, 108; 106', 108') deflect measuring radiation incident on their incidence region (84a, 84b, 84c, 84d) to a different extent, respectively, relative to an optical axis (V) of the beam splitter arrangement (84; 84').

3. Radiation sensor assembly (54) according to Claim 1 or 2,
**characterized in that** the first incidence region (84a) comprises a plurality of first and/or of second deflection zones (106, 108; 106', 108')
and/or
That the second incidence region (84b) comprises a plurality of first and/or of second deflection zones (106, 108; 106', 108').

4. Radiation sensor assembly (54) according to one of the Claims 1 to 3,
**characterized in that** the first and the second deflection zone (106', 108') of an incidence region (84a, 84b, 84c, 84d) differ from one another through locally different interface shapes at an interface (85') separating the beam splitter arrangement from its environment in the region of its incidence region (84a, 84b, 84c, 84d), wherein an interface region exhibiting the first deflection zone (106') and the second deflection zone (108') exhibits a surface envelope (OH') whose distance (d) from a tangential plane (TE') to the interface (85') orthogonal to the optical axis (V), to be measured parallel to an optical axis (V) of the beam splitter arrangement (84'), increases with increasing distance from the optical axis (V) of the beam splitter arrangement (84').

5. Radiation sensor assembly (54) according to Claim 4,
**characterized in that** a refractively active section of the beam splitter arrangement (84; 84') is configured as mirror-symmetrical, in particular with respect to a symmetry plane (SE1, SE2) containing the optical axis (V).

6. Radiation sensor assembly (54) according to one of the Claims 4 or 5,
**characterized in that** a refractively active section of the beam splitter arrangement (84; 84') is configured rotation-symmetrically relative to the optical axis (V) as the rotational symmetry axis.

7. Radiation sensor assembly (54) according to one of the preceding Claims, **characterized in that** at least one deflection zone of the at least one first deflection zone (106; 106') of the first (84a) and of the second incidence region (84b) is at least section-wise, preferably completely, configured as integrally connected and/or at least one deflection zone of the at least one second deflection zone (108; 108') of the first (84a) and of the second incidence region (84b) is at least section-wise, preferably completely, configured as integrally connected.

8. Radiation sensor assembly (54) according to one of the preceding Claims, **characterized in that** the radiation sensor assembly (54) further comprises:
- a third bandpass filter (98) arranged spatially distant from the first (90) and from the second bandpass filter (94), reachable by a third part of the measuring radiation, with a predetermined third bandwidth and with a transmission maximum at a predetermined second useful signal wavelength,
- a second measuring radiation useful signal sensor (100) arranged spatially distant from the first measuring radiation useful signal sensor (92) and from the first measuring radiation reference signal sensor (96), arranged in the beam path behind the third bandpass filter (98), on which the measuring radiation traversing the third bandpass filter (98) is incident,
- a fourth bandpass filter (102) arranged spatially distant from the first (90), second (94), and third bandpass filter (98), which is reachable by a fourth part of the measuring radiation which is different from the first, second, and third part, wherein the fourth bandpass filter (102) exhibits a predetermined fourth bandwidth and a transmission maximum at a predetermined second reference signal wavelength, wherein the second reference signal wavelength differs from the second useful signal wavelength,
- a second measuring radiation reference signal sensor (104) arranged in the beam path behind the fourth bandpass filter (102) and spatially distant from the first (92) and second measuring radiation useful signal sensor (100) and from the first measuring radiation reference signal sensor (96), on which the measuring radiation traversing the fourth bandpass filter (102) is incident,
wherein the beam splitter arrangement (84; 84') exhibits a third incidence region (84c) spatially different from the first (84a) and from the second incidence region (84b) and a fourth incidence region (84d) spatially different from the first (84a), second (84b), and third incidence region (84c), wherein in the third and fourth incidence regions (84c, 84d) measuring radiation is incident on the beam splitter arrangement (84; 84'), and wherein the third (84c) and the fourth incidence region (84d) are optically configured in such a way that
- the beam splitter arrangement (84; 84') in the third incidence region (84c)
+ deflects a first part of the measuring radiation incident on the third incidence region (84c) onto the third bandpass filter (98), and
+ deflects a second part of the measuring radiation incident on the third incidence region (84c) onto the fourth bandpass filter (102),
and that
- the beam splitter arrangement (84; 84') in the fourth incidence region (84d)
+ deflects a first part of the measuring radiation incident on the fourth incidence region (84d) onto the fourth bandpass filter (102), and
+ deflects a second part of the measuring radiation incident on the fourth incidence region (84d) onto the third bandpass filter (98).

9. Radiation sensor assembly (54) according to one of the preceding Claims,
**Characterized in that** a plurality of bandpass filters (90, 94, 98, 102) and/or a plurality of measuring radiation sensors (92, 96, 100, 104) are each arranged in a bandpass filter arrangement plane (BA) and/or in a sensor arrangement plane (SA) respectively.

10. Radiation sensor assembly (54) according to one of the preceding Claims, **characterized in that** it exhibits at a distance from the beam splitter arrangement (84; 84') a measuring radiation source (74), which is configured to emit measuring radiation towards the beam splitter arrangement (84; 84').

11. Radiation sensor assembly (54) according to Claim 10,
**characterized in that** the measuring radiation source (74) is configured to emit collimated measuring radiation.

12. Radiation sensor assembly (54) according to one of the preceding Claims, **characterized in that** the radiation sensor assembly (54) comprises an evaluation device (14, 76) which from the signals of the first measuring radiation reference signal sensor (96) obtains reference information, which from the signals of the first measuring radiation useful signal sensor (92) obtains useful information, and which from a comparison of the reference information and useful information outputs information about a fraction of a gas component of a measuring gas exposed to the measuring radiation identified by means of the first useful signal wavelength.

13. Radiation sensor assembly (54) according to one of the preceding Claims, by reference to Claim 10,
**Characterized in that** the radiation sensor assembly (54) exhibits a sensor housing (67) with a first compartment (68), at or in which the beam splitter arrangement (84; 84'), the measuring radiation useful signal sensor (92), and the measuring radiation reference signal sensor (96) are arranged, and with a second compartment (72) located spatially distant rom the first compartment (68), at or in which the measuring radiation source (74) is arranged, wherein between the first (68) and the second compartment (72) there is arranged an accommodating formation (79) for accommodating a measuring cuvette (52) between the first (68) and the second compartment (72).

14. Ventilation device (10) for at least supportive artificial ventilation of a living patient, comprising:
- a respiratory gas source (12),
- a ventilation line arrangement (20), in order to conduct inspiratory respiratory gas from the respiratory gas source (12) to a patient-side, proximal respiratory gas outlet aperture (62) and in order to conduct expiratory respiratory gas away from a proximal respiratory gas inlet aperture (62),
- a pressure-changing device (13) for changing the pressure of the respiratory gas in the ventilation line arrangement (20),
- a control device (14) for operating the respiratory gas source (12) and/or the pressure-changing device (13), and
- a multichannel radiation sensor assembly (54) according to one of the preceding Claims for detecting at least one gas component in the inspiratory and/or expiratory respiratory gas.

## Revendications

1. Ensemble de capteur de rayonnement non dispersif à canaux multiples (54) pour la détermination quantitative d'une partie d'un fluide de mesure absorbant un rayonnement de mesure électromagnétique, comprenant :
- un ensemble de diviseur de faisceau (84, 84') qui est conçu pour diviser un faisceau (64) du rayonnement de mesure qui est incident sur l'ensemble de diviseur de faisceau (84, 84') le long d'un axe d'incidence (E) prédéterminé,
- un premier filtre passe-bande (90) pouvant être atteint par une première partie (64a, 64c) du rayonnement de mesure, avec une première largeur de bande prédéterminée et avec un maximum de transmission à une première longueur d'onde de signal utile prédéterminée,
- un premier capteur de signal utile de rayonnement de mesure (92) disposé dans le trajet du faisceau derrière le premier filtre à bande (90), sur lequel le rayonnement de mesure traversant le premier filtre à bande (90) est incident,
- un deuxième filtre à bande (94) disposé spatialement à distance du premier filtre à bande (90), qui peut être atteint par une deuxième partie (64b, 64d) du rayonnement de mesure différente de la première partie (64a, 64c), dans lequel le deuxième filtre à bande (94) présente une deuxième largeur de bande prédéterminée et un maximum de transmission à une première longueur d'onde de signal de référence prédéterminée, dans lequel la première longueur d'onde de signal de référence est différente de la première longueur d'onde de signal utile,
- un premier capteur de signal de référence de rayonnement de mesure (96) disposé dans le trajet du faisceau derrière le deuxième filtre à bande (94) et éloigné dans l'espace du premier capteur de signal utile de rayonnement de mesure (92), sur lequel le rayonnement de mesure traversant le deuxième filtre à bande (94) est incident,
**caractérisé en ce que** l'ensemble de diviseur de faisceau (84, 84') est un ensemble de diviseur de faisceau (84, 84') traversé par le rayonnement de mesure et réfractant le rayonnement de mesure qui le traverse, qui présente une première zone de rayonnement (84a) et une deuxième zone de rayonnement (84b) différente dans l'espace de la première, dans lesquelles zones de rayonnement (84a, 84b), un rayonnement de mesure est émis sur l'ensemble de diviseur de faisceau (84, 84'), dans lequel la première (84a) et la deuxième (84b) zones de rayonnement sont réalisées optiquement de telle sorte que
- l'ensemble de diviseur de faisceau (84, 84') dans la première zone de rayonnement (84a)
+ dévie une première partie (64a) du rayonnement de mesure rayonnant sur la première zone de rayonnement (84a) sur le premier filtre à bande (90), et
+ dévie une deuxième partie (64c) du rayonnement de mesure rayonnant sur la première zone de rayonnement incident (84a) sur le deuxième filtre à bande (94),
et **en ce que**
- l'ensemble de diviseur de faisceau (84, 84') dans la deuxième zone de rayonnement (84b)
+ dévie une première partie (64b) du rayonnement de mesure rayonnant sur la deuxième zone de rayonnement (84b) sur le deuxième filtre à bande (94) et
+ dévie une deuxième partie (64d) du rayonnement de mesure rayonnant sur la deuxième zone de rayonnement (84b) sur le premier filtre à bande (90),
dans lequel la première zone de rayonnement (84a) est optiquement réfractive et présente au moins deux zones de déviation (106, 108 ; 106', 108') déviant le rayonnement de mesure rayonnant respectivement dans des directions différentes, dans lequel une première zone de déviation (106 ; 106') provoque la déviation de la première partie (64a) du rayonnement de mesure incident sur la première zone de rayonnement (84a) sur le premier filtre à bande (90) et dans lequel une deuxième zone de déviation (108 ; 108') provoque la déviation de la deuxième partie (64c) du rayonnement de mesure incident sur la première zone de rayonnement (84a) sur le deuxième filtre à bande (94),
ou/et
la deuxième zone de rayonnement (84b) est optiquement réfractive et comporte au moins deux zones de déviation (106, 108 ; 106", 108'), dans lequel une première zone de déviation provoque la déviation de la première partie (64b) du rayonnement de mesure rayonnant sur la deuxième zone de rayonnement (84b) et dans lequel une deuxième zone de déviation (108 ; 108') provoque la déviation de la deuxième partie (64d) du rayonnement de mesure rayonnant sur la deuxième zone de rayonnement (84b), dans lequel la première et la deuxième zone de déviation (106, 108 ; 106', 108') d'une zone de rayonnement (84a, 84b, 84c, 84d) se distinguent l'une de l'autre par des matériaux différents utilisés pour leur réalisation respective et présentent des indices de réfraction différents et/ou par des configurations d'interface localement différentes au niveau d'une interface (85 ; 85') séparant l'ensemble de diviseur de faisceau (84 ; 84') de son environnement dans la zone de sa zone de rayonnement (84a, 84b, 84c, 84d).

2. Ensemble de capteur de rayonnement (54) selon la revendication 1,
**caractérisé en ce que** la première et la deuxième zone de déviation (106, 108 ; 106', 108') dévient le rayonnement de mesure incident sur leur zone de rayonnement (84a, 84b, 84c, 84d) avec une intensité différente par rapport à un axe optique (V) de l'ensemble de diviseur de faisceau (84 ; 84').

3. Ensemble de capteur de rayonnement (54) selon la revendication 1 ou 2, **caractérisé en ce que** la première zone de rayonnement (84a) comprend une pluralité de premières ou/et de deuxièmes zones de déviation (106, 108 ; 106', 108') ou/et **en ce que** la deuxième zone de rayonnement (84b) comprend une pluralité de premières ou/et de deuxièmes zones de déviation (106, 108 ; 106', 108').

4. Ensemble de capteur de rayonnement (54) selon l'une des revendications 1 à 3,
**caractérisé en ce que** la première et la deuxième zone de déviation (106', 108') d'une zone de rayonnement (84a, 84b, 84c, 84d) se distinguent l'une de l'autre par des formes de surface limite localement différentes sur une surface limite (85') séparant l'ensemble de diviseur de faisceau de son environnement dans la zone de sa zone de rayonnement (84a, 84b, 84c, 84d), dans lequel une zone de surface limite présentant la première zone de déviation (106') et la deuxième zone de déviation (108') présente une enveloppe de surface (OH') dont la distance (d) à mesurer parallèlement à un axe optique (V) de l'ensemble de diviseur de faisceau (84') à partir d'un plan tangentiel (TE') orthogonal à l'axe optique (V) à la surface limite (85') augmente avec un éloignement croissant de l'axe optique (V) de l'ensemble de diviseur de faisceau (84').

5. Ensemble de capteur de rayonnement (54) selon la revendication 4,
**caractérisé en ce qu'**une section à effet réfractif de l'ensemble de diviseur de faisceau (84 ; 84') est réalisée avec une symétrie de miroir, notamment par rapport à un plan de symétrie (SE1, SE2) contenant l'axe optique (V).

6. Ensemble de capteur de rayonnement (54) selon l'une des revendications 4 ou 5,
**caractérisé en ce qu'**une section à effet réfractif de l'ensemble de diviseur de faisceau (84 ; 84') est réalisée avec une symétrie de rotation par rapport à l'axe optique (V) en tant qu'axe de symétrie de rotation.

7. Ensemble de capteur de rayonnement (54) selon l'une des revendications précédentes,
**caractérisé en ce qu'**au moins une zone de déviation de ladite au moins une première zone de déviation (106 ; 106') de la première (84a) et de la deuxième zone de rayonnement (84b) est réalisée au moins en partie, de préférence entièrement, de manière continue d'une seule pièce ou/et au moins une zone de déviation de ladite au moins une deuxième zone de déviation (108 ; 108') de la première (84a) et de la deuxième zone de rayonnement (84b) est réalisée au moins en partie, de préférence entièrement, de manière continue d'une seule pièce.

8. Ensemble de capteur de rayonnement (54) selon l'une des revendications précédentes,
**caractérisé en ce que** l'ensemble de capteur de rayonnement (54) comprend en outre :
- un troisième filtre à bande (98), éloigné spatialement du premier (90) et du deuxième filtre à bande (94) et accessible par une troisième partie du rayonnement de mesure, avec une troisième largeur de bande prédéterminée et avec un maximum de transmission à une deuxième longueur d'onde de signal utile prédéterminée,
- un deuxième capteur de signal utile de rayonnement de mesure (100) disposé à distance du premier capteur de signal utile de rayonnement de mesure (92) et du premier capteur de signal de référence de rayonnement de mesure (96), dans le trajet du faisceau derrière le troisième filtre à bande (98), sur lequel le rayonnement de mesure traversant le troisième filtre à bande (98) est incident,
- un quatrième filtre à bande (102) disposé spatialement à distance du premier (90), du deuxième (94) et du troisième filtre à bande (98), lequel peut être atteint par une quatrième partie du rayonnement de mesure différente des première, deuxième et troisième parties, dans lequel le quatrième filtre à bande (102) présente une quatrième largeur de bande prédéterminée et un maximum de transmission à une deuxième longueur d'onde de signal de référence prédéterminée, dans lequel la deuxième longueur d'onde de signal de référence est différente de la deuxième longueur d'onde de signal utile,
- un deuxième capteur de signal de référence de rayonnement de mesure (104) disposé dans le trajet du faisceau derrière le quatrième filtre à bande (102) et éloigné dans l'espace du premier (92) et du deuxième capteur de signal utile de rayonnement de mesure (100) ainsi que du premier capteur de signal de référence de rayonnement de mesure (96), sur lequel le rayonnement de mesure traversant le quatrième filtre à bande (102) est incident,
dans lequel l'ensemble de diviseur de faisceau (84 ; 84') présente une troisième zone de rayonnement (84c) différente dans l'espace de la première (84a) et de la deuxième (84b) zone de rayonnement ainsi qu'une quatrième zone de rayonnement (84d) différente dans l'espace de la première (84a), de la deuxième (84b) et de la troisième (84c) zone de rayonnement, dans lequel un rayonnement de mesure est appliqué sur l'ensemble de diviseur de faisceau (84 ; 84') dans les troisième et quatrième zones de rayonnement (84c, 84d), et dans lequel la troisième (84c) et la quatrième zone de rayonnement (84d) sont conçues optiquement de telle sorte que
- l'ensemble de diviseur de faisceau (84 ; 84') dans la troisième zone de rayonnement (84c)
+ dévie une première partie du rayonnement de mesure incident sur la troisième zone de rayonnement (84c) sur le troisième filtre à bande (98), et
+ dévie une deuxième partie du rayonnement de mesure incident sur la troisième zone de rayonnement (84c) sur le quatrième filtre à bande (102),
et **en ce que**
- l'ensemble de diviseur de faisceau (84 ; 84') dans la quatrième zone de rayonnement (84d)
+ dévie une première partie du rayonnement de mesure incident sur la quatrième zone de rayonnement (84d) sur le quatrième filtre à bande (102) et
+ dévie une deuxième partie du rayonnement de mesure incident sur la quatrième zone de rayonnement (84d) sur le troisième filtre à bande (98).

9. Ensemble de capteur de rayonnement (54) selon l'une des revendications précédentes,
**caractérisé en ce qu'**une pluralité de filtres à bande (90, 94, 98, 102) ou/et une pluralité de capteurs de rayonnement de mesure (92, 96, 100, 104) sont respectivement disposés dans un plan de disposition des filtres à bande (BA) ou dans un plan de disposition de capteurs (SA).

10. Ensemble de capteur de rayonnement (54) selon l'une des revendications précédentes,
**caractérisé en ce qu'**il présente, à distance de l'ensemble de diviseur de faisceau (84 ; 84'), une source de rayonnement de mesure (74) qui est conçue pour émettre un rayonnement de mesure vers l'ensemble de diviseur de faisceau (84 ; 84').

11. Ensemble de capteur de rayonnement (54) selon la revendication 10,
**caractérisé en ce que** la source de rayonnement de mesure (74) est conçue pour émettre un rayonnement de mesure collimaté.

12. Ensemble de capteur de rayonnement (54) selon l'une des revendications précédentes,
**caractérisé en ce que** l'ensemble de capteur de rayonnement (54) comprend un ensemble d'évaluation (14, 76) qui détermine une information de référence à partir des signaux du premier capteur de signal de référence de rayonnement de mesure (96), qui détermine une information utile à partir des signaux du premier capteur de signal utile de rayonnement de mesure (92), et qui délivre, à partir d'une comparaison entre l'information de référence et l'information utile, une information sur une proportion d'une partie de gaz identifiée par la première longueur d'onde de signal utile dans un gaz de mesure irradié par le rayonnement de mesure.

13. Ensemble de capteur de rayonnement (54) selon l'une des revendications précédentes, y compris la revendication 10,
**caractérisé en ce que** l'ensemble de capteur de rayonnement (54) comprend un boîtier de capteur (67) avec un premier compartiment (68) sur ou dans lequel l'ensemble de diviseur de faisceau (84 ; 84'), le capteur de signal utile de rayonnement de mesure (92) et le capteur de signal de référence de rayonnement de mesure (96) sont disposés, et avec un deuxième compartiment (72) situé spatialement à distance du premier compartiment (68), sur ou dans lequel est disposée la source de rayonnement de mesure (74), dans lequel une formation de réception (79) est disposée entre le premier (68) et le deuxième compartiment (72) pour recevoir une cuvette de mesure (52) entre le premier (68) et le deuxième compartiment (72).

14. Ensemble de ventilation (10) pour la ventilation artificielle au moins en assistance d'un patient vivant, comprenant :
- une source de gaz respiratoire (12),
- un ensemble de conduit de ventilation (20) pour diriger le gaz de ventilation inspiratoire de la source de gaz de ventilation (12) vers un orifice de sortie de gaz de ventilation proximal (62) du côté du patient et pour éloigner le gaz de ventilation expiratoire d'un orifice d'entrée de gaz de ventilation proximal (62),
- un ensemble de modification de la pression (13) pour modifier la pression du gaz respiratoire dans l'ensemble de conduits respiratoires (20),
- un ensemble de commande (14) pour l'opération de la source de gaz respiratoire (12) ou/et de l'ensemble de modification de la pression (13) ainsi que
- un ensemble de capteurs de rayonnement à canaux multiples (54) selon l'une des revendications précédentes pour détecter au moins un composant gazeux dans le gaz respiratoire inspiratoire ou/et expiratoire.
